# EUROPÄISCHE PATENTSCHRIFT

(11) **EP 0 971 751 B1**
(45) Veröffentlichungstag und Bekanntmachung des Hinweises auf die Patenterteilung: **21.11.2001**
(21) Anmeldenummer: 98917054.3
(22) Anmeldetag: 23.03.1998
(51) Int. Cl.: A61L 15/60, A61F 13/15

(54) **ABSORBIERENDER ARTIKEL**
ABSORBENT ITEM
ARTICLE ABSORBANT

(30) Priorität: 27.03.1997 DE 19713189; 27.03.1997 DE 19713190; 17.02.1998 DE 19806575
(43) Veröffentlichungstag der Anmeldung: 19.01.2000
(73) Patentinhaber: Kimberly-Clark GmbH, 56070 Koblenz (DE)
(72) Erfinder: RAIDEL, Maria, D-90451 Nürnberg (DE); ASCHENBRENNER, Franz, D-92280 Kastl (DE)
(74) Vertreter: Diehl, Hermann, Dr.
(86) Internationale Anmeldenummer: EP9801684
(87) Internationale Veröffentlichungsnummer: WO9843684

(56) Entgegenhaltungen:
- EP-A- 0 297 411
- EP-A- 0 341 951
- DE-U- 8 815 855
- GB-A- 2 211 418
- US-A- 3 532 097
- US-A- 3 585 998
- US-A- 4 401 795
- US-A- 4 587 308
- US-A- 5 147 921

## Beschreibung

Die vorliegende Erfindung betrifft absorbierende Artikel.

Absorbierende Artikel zum einmaligen Gebrauch sind seit vielen Jahren bekannt. Sie finden beispielsweise als Damenbinden, Slipeinlagen, Kinderwindeln oder Inkontinenzeinlagen Verwendung. Diesen absorbierenden Wegwerf-Artikeln ist gemeinsam, daß sie eine beim Tragen dem Körper des Trägers zugewandte, flüssigkeitsdurchlässige Schicht, eine beim Tragen dem Körper des Trägers abgewandte, flüssigkeitsundurchlässige Schicht, sowie eine zwischen diesen beiden Schichten angeordnete flüssigkeitsspeichernde Schicht aufweisen. Die flüssigkeitsspeichernde Schicht kann dabei zum Beispiel aus zerfasertem Zellstoff aufgebaut sein.

Als nachteilig bei den bekannten absorbierenden Wegwerfartikeln hat sich erwiesen, daß reine Zellstoffschichten als flüssigkeitsspeicherndes Material von der Aufnahmekapazität her beschränkt sind. Auch ist das Rückhaltevermögen nach Beaufschlagung mit Flüssigkeiten bei einem Zellstoffmaterial nicht sehr ausgeprägt. Schließlich behält deformiertes Zellstoffmaterial seine einmal angenommene Form bei, was vom Träger bzw. der Trägerin des absorbierenden Artikels oft als unangenehm empfunden wird. Wenn im folgenden vom Träger die Rede ist, soll dies sowohl Träger als auch Trägerinnen umfassen.

Des weiteren sind absorbierende Artikel zum Einmalgebrauch bekannt, welche als flüssigkeitsspeichernde Schicht superabsorbierende Materialien enthalten. Superabsorbierende Materialien sind in der Lage, ein Vielfaches ihres Trockengewichts an Flüssigkeit aufzunehmen und auch unter Druckbelastung in einem gewissen Maße zurückzuhalten.

Superabsorbierende Materialien sind beispielsweise aus der EP-A-0339461 bekannt.

Eine Schwierigkeit bei der Verwendung von superabsorbierenden Materialien in der flüssigkeitsspeichernden Schicht von absorbierenden Artikeln ist, daß die superabsorbierenden Materialien bei Beaufschlagung mit Flüssigkeit eine Volumenzunahme erfahren, d.h. sie "quellen". Dies führt dazu, daß der absorbierende Artikel "aufträgt" und der Tragekomfort für den Träger herabgesetzt wird. Auch neigen die einzelnen Bestandteile des superabsorbierenden Materials dazu, bei Beaufschlagung mit einer Flüssigkeit zu verkleben, was zu einer starken Verminderung des theoretisch möglichen Flüssigkeitsaufnahmevermögens führt (sog. "Gel-Blocking"). "Gel-Blocking" verursacht eine eingeschränkte Verteilung der in den absorbierenden Artikel eingedrungenen Flüssigkeit. Bei starker Flüssigkeitsbeaufschlagung kann dies zur Folge haben, daß die Flüssigkeit nicht mehr vollständig in den absorbierenden Artikel aufgenommen werden kann, obwohl theoretisch noch ausreichend Speicherkapazität zur Verfügung stünde, wobei sich beim Träger des Artikels ein Nässe- und damit Unsauberkeitsgefühl auf der Haut einstellt und darüberhinaus auch die Gefahr besteht, daß die Bekleidung des Trägers verschmutzt wird. Schließlich kann der absorbierende Artikel durch den "Gel-Blocking"-Effekt auch bleibend verformt werden, wodurch der Tragekomfort des Artikels weiter vermindert wird.

Es ist somit die Aufgabe der vorliegenden Erfindung, absorbierende Artikel anzugeben, welche die oben beschriebenen Nachteile bekannter Produkte nicht aufweisen. Diese Aufgabe löst die Erfindung durch den in den unabhängigen Patentansprüchen 1 und 2 angegebenen absorbierenden Artikel. Weitere vorteilhafte Ausgestaltungen, Details und Aspekte der vorliegenden Erfindung ergeben sich aus den abhängigen Patentansprüchen, der Beschreibung und den Zeichnungen.

Die vorliegende Erfindung betrifft gemäß einem ersten Aspekt einen absorbierenden Artikel, welcher eine dem Körper des Trägers zugewandte, flüssigkeitsdurchlässige Schicht und eine dem Körper des Trägers abgewandte, flüssigkeitsundurchlässige Schicht aufweist. Zwischen diesen beiden Schichten ist ein Saugkörper angeordnet, welcher eine durch die flüssigkeitsdurchlässige Schicht in den absorbierenden Artikel eingedrungene Flüssigkeit aufnimmt. Der Saugkörper des erfindungsgemäßen absorbierenden Artikels ist dadurch ausgezeichnet, daß dieser ein saugfähiges Material enthält, welches auch nach Beaufschlagung mit einer Flüssigkeit rieselfähig bleibt. Durch diese neue Ausgestaltung wird einerseits eine optimale Anpassung des absorbierenden Artikels an die individuelle Körperform des Trägers erreicht, was den Tragekomfort erheblich erhöht. Andererseits wird mit der erfindungsgemäßen Lösung aber auch erreicht, daß die Funktionalität des Artikels auch in einem deformierten Zustand aufrechterhalten wird.

Eine spezielle Ausgestaltung des erfindungsgemäßen absorbierenden Artikels gemäß des ersten Aspekts stellt eine Ausführungsform dar, bei welcher die flüssigkeitsdurchlässige obere Abdeckschicht in Längsrichtung verlaufende Falten aufweist. Diese Falten sind derart ausgestaltet, daß die flüssigkeitsdurchlässige Schicht den Saugkörper teilweise auch auf dessen der beim Tragen des Artikels dem Körper abgewandten Seite umhüllt. Dies wird zum einen dadurch erreicht, daß der Saugkörper nicht auf seiner gesamten "Unterseite" mit der darunterliegenden Schicht verbunden ist, sondern nur in einem schmalen, zentralen Bereich. Zum anderen werden in die flüssigkeitsdurchlässige Schicht zwei Falten eingearbeitet, welche den Saugkörper in Längsrichtung des absorbierenden Artikels auch teilweise unterseitig umgreifen.

Durch diese Ausgestaltung wird erreicht, daß der Saugkörper trotz Einbettung zwischen flüssigkeitsundurchlässiger Rückschicht und flüssigkeitsdurchlässiger Abdeckschicht flexibel bleibt und sich den anatomischen Gegebenheiten des Trägers sehr gut anpassen kann.

Wenn die flüssigkeitsdurchlässige und die flüssigkeitsundurchlässige Schicht in deren Randbereichen derart miteinander verbunden sind, daß ein dicht abgeschlossener Innenraum entsteht, so kann der Saugkörper von einem lose, verschiebbar gegeneinander gelagerten Material gebildet werden, welches auch nach Flüssigkeitsbeaufschlagung rieselfähig verbleibt, wobei das Material sich frei in dem gesamten Innenraum bewegen kann.

Gemäß einem weiteren Aspekt betrifft die Erfindung einen absorbierenden Artikel mit einer bei Verwendung des Artikels dem Körper abgewandten, flüssigkeitsundurchlässigen Schicht und einem von einer flüssigkeitsdurchlässigen Umhüllung umgebenen Saugkörper, welcher ein auch nach Beaufschlagung mit Flüssigkeit rieselfähig bleibendes, saugfähiges Material enthält, wobei der Saugkörper mit der flüssigkeitsundurchlässigen Schicht in einem zentralen Bereich derselben verbunden ist. Dabei ist es nicht notwendig, daß der Saugkörper und die flüssikeitsundurchlässige Schicht unmittelbar in Kontakt stehen. Wenn auf der flüssigkeitsundurchlässigen Schicht noch eine oder mehrere andere Schichten angeordnet sind, ist der Saugkörper auf der Oberseite der obersten dieser Schichten befestigt.

Ein weiterer Aspekt der Erfindung betrifft einen absorbierenden Artikel, dessen Saugkörper aus einem saugfähigen Material besteht, welches Polymethylenharnstoff (PMH) ist. Der absorbierende Artikel kann eine bei Verwendung des Artikels dem Körper zugewandte, flüssigkeitsdurchlässige Schicht sowie eine bei Verwendung des Artikels dem Körper abgewandte, flüssigkeitsundurchlässige Schicht aufweisen, wobei zwischen der flüssigkeitsdurchlässigen und der flüssigkeitsundurchlässigen Schicht der Saugkörper angeordnet ist. Es sind aber auch alternative Ausgestaltungen denkbar, welche entsprechend dem vorstehend beschriebenen Aspekt ohne flüssigkeitsdurchlässige obere Abdeckschicht auskommen und stattdessen nur das PMH-Material in einer flüssigkeitsdurchlässigen Umhüllung einbetten.

Das neue Konzept weicht von bekannten Konstruktionen dadurch ab, daß ein Anteil, vorzugsweise der Hauptanteil, der saugenden Bestandteile eine körnige, möglichst aus kugelförmig ausgebildeten Materialien aufgebaute Beschaffenheit aufweist. Das Saugkörpermaterial wird derartig zusammengesetzt, daß dieses während des Tragens und auch bei Beaufschlagung mit Flüssigkeit rieselfähig bleibt. Vorzugsweise bleibt das Saugkörpermaterial bis zu einer Flüssigkeitsbeaufschlagung von mindestens 10 ml/g Material rieselfähig. Dadurch wird eine optimale Anpassung ("Anschmiegsamkeit") an die jeweilige Körperform des Trägers während der unterschiedlichen Bewegungen und Belastungsarten ermöglicht. Das heißt, der Saugkörper "fließt", er kann bei seitlicher Belastung bzw. seitlichem Druck durch die Oberschenkel dieser Belastung bzw. diesem Druck etwas ausweichen, indem Saugkörpermaterial in weniger beanspruchte Bereiche verlagert oder verdrängt wird. Bei Entfernung der Belastung bzw. des Drucks können verdrängte Partikel an die Ausgangsstelle zurückrieseln und erneut zur Aufnahme von Flüssigkeit zur Verfügung stehen. Andererseits können durch diese Bewegungen auch Teilchen repositioniert werden und somit bisher ungenutzte Saugkapazität und Speicherkapazität genutzt werden. Sofern im vorliegenden Fall von "Saugkörper" die Rede ist, ist damit auch ein "Speicherkörper" gemeint.

Durch den Saugkörper, der gleichzeitig als Speicherschicht dient und ein saugfähiges Material enthält, welches auch nach einer Beaufschlagung mit einer Flüssigkeit rieselfähig bleibt, ergeben sich für die erfindungsgemäßen absorbierenden Artikel folgende vorteilhafte Eigenschaften:
- Schnelle Flüssigkeitsaufnahme (gute Penetration in das rieselfähige Material und gute Benetzung des Materials),
- gute Retention der Flüssigkeit (Einschließen der Flüssigkeit auch unter Druckbelastung),
- gute Saugleistung (Absorbtion praktisch ohne Volumenzunahme),
- Verhinderung von Verklumpungen bei Flüssigkeitsbeaufschlagung,
- bestmögliche individuelle Körperanpassung,
- hohe Weichheit des Artikels verbunden mit großem Tragekomfort,
- sehr guter Flüssigkeitstransport und gute Flüssigkeitsverteilung,
- kein Kollabieren bzw. "Sumpfen", wie es bei Zellstoffsaugkörpern vorkommt.

Besonders bei dem erfindungsgemäßen absorbierenden Artikel gemäß dem vorgenannten weiteren Aspekt ergibt sich beim Tragen des Artikels eine optimale Anpassung an anatomische Gegebenheiten, da der umhüllte Saugkörper praktisch "freiliegt", d.h., nicht von einer den ganzen Artikel überspannenden, flüssigkeitsdurchlässigen Schicht vom Körper des Trägers getrennt ist. Die aus dem Körper austretende Flüssigkeit kann unmittelbar an der Austrittsstelle in den Saugkörper aufgenommen und dort weitergeleitet bzw. gespeichert werden.

Die Verbindung zwischen der flüssigkeitsundurchlässigen Rückschicht und dem von einer flüssigkeitsdurchlässigen Schicht umhüllten Saugkörper kann auf jede geeignete Art erfolgen. Als günstig bei der maschinellen Herstellung des erfindungsgemäßen Artikels hat sich beispielsweise eine durch ein Haftmittel bewirkte Verbindung erwiesen. Die Rückschicht und der Saugkörper können aber auch beispielsweise durch Vernähung fest miteinander verbunden werden, wobei natürlich darauf zu achten ist, daß die flüssigkeitsundurchlässige Rückschicht nicht derart beschädigt wird, daß Flüssigkeit hindurchtreten kann.

Des weiteren hat es sich als vorteilhaft herausgestellt, wenn die erfindungsgemäßen absorbierenden Artikel eine auf der dem Körper des Trägers zugewandten Seite der flüssigkeitsundurchlässigen Schicht weitere Schicht aus weichem Material, das als Sekundärspeicher dienen kann, aufweisen. Diese weitere Schicht erhöht den Tragekomfort des absorbierenden Artikels zusätzlich. Darüber hinaus kann diese weitere Schicht auch noch nicht vom Hauptsaugkörper aufgenommene Flüssigkeit speichern, wobei natürlich das absolute Speichervermögen der weiteren Schicht im Vergleich zum eigentlichen Saugkörper sehr viel geringer ist. Geeignete Materialien für die weitere Schicht sind Coform (Polypropylen-Zellstoff-Mischungen), Airlaid (Kunstfaser-Zellstoff-Mischungen) und Vliesmaterialien, beispielsweise Spinnvliese oder Kardenvliese.

Gemäß eines weiteren Aspekts der vorliegenden Erfindung kann das saugfähige Material, welches auch nach Beaufschlagung mit einer Flüssigkeit rieselfähig bleibt, in einer Matrix aus Fasermaterial eingelagert sein. Dabei kann das Material homogen in die Fasermatrix eingemischt werden, so daß die Bestandteile des Materials, welches auch nach Beaufschlagung mit einer Flüssigkeit rieselfähig bleibt, gleichmäßig über die Faserstruktur verteilt und in dieser eingelagert sind. Alternativ dazu kann der Saugkörper aber auch einen schichtartigen Aufbau zeigen, wobei das saugfähige Material, welches auch nach Beaufschlagung mit einer Flüssigkeit rieselfähig bleibt, sandwichartig zwischen zwei oder mehreren Schichten aus Fasermaterial eingebettet ist. Schließlich kann bei der zuletzt beschriebenen Sandwichstruktur in den Faserschichten auch noch zusätzlich saugfähiges Material, welches auch nach Beaufschlagung mit einer Flüssigkeit rieselfähig bleibt, eingelagert sein. Materialien, welche als Faserstoffe für die vorgenannten Zwecke besonders geeignet sind, sind Zellstoff oder eine Mischung aus Zellstoff und Polypropylen, d.h. ein sogenanntes Coform-Material. Durch das Fasermaterial wird eine noch optimalere Flüssigkeitsverteilung in dem erfindungsgemäßen absorbierenden Artikel erreicht, da die Fasern eine bestimmte Saugfähigkeit aufweisen und Flüssigkeit gerichtet transportieren können. Das Verhältnis von saugfähigem Material, welches auch nach Beaufschlagung mit einer Flüssigkeit rieselfähig bleibt, und Fasermaterial beträgt vorzugsweise 1 bis 25 Gew.-% zu 99 bis 75 Gew.-% und insbesondere 10 bis 15 Gew.-% zu 90 bis 85 Gew.-%.

Gemäß eines weiteren Aspekts der Erfindung kann der Saugkörper neben dem saugfähigen Material, welches auch nach Beaufschlagung mit einer Flüssigkeit rieselfähig bleibt, mindestens eine pflegende Substanz adsorptiv gebunden enthalten. Hierbei ist in erster Linie an Substanzen gedacht, welche die Haut des Trägers des erfindungsgemäßen absorbierenden Artikels schützen. Geeignete Substanzen sind beispielsweise Extrakte aus Aloe Vera, Ringelblumen (Calendula) und/oder Kamille (Matricaria).

Von besonderem Vorteil ist es, wenn die pflegenden Substanzen in Mikrokapseln eingeschlossen sind. Die Mikrokapseln können dabei mit dem saugfähigen Material, welches auch nach Beaufschlagen mit einer Flüssigkeit rieselfähig bleibt, vermischt werden. Die Umhüllung der Mikrokapseln sollte dabei so ausgestaltet sein, daß diese beim Tragen des erfindungsgemäßen absorbierenden Artikels aufplatzt und die Substanz bzw. die Substanzen freigibt. Das Aufplatzen kann beispielsweise durch Druck, Wärme und/oder Reibung bewirkt werden. Die Mikroverkapselung von Substanzen ist beispielsweise in der Drucktechnik schon seit längerem bekannt.

Ein besonders geeignetes Material, welches im Saugkörper bzw. Flüssigkeitsspeicher des erfindungsgemäßen absorbierenden Artikels einsetzbar ist, ist Polymethylenharnstoff (PMH) mit einer partikulären Struktur. PMH bleibt auch nach Beaufschlagung mit einer Flüssigkeit, wie insbesondere Urin oder Menstruationsblut, rieselfähig. Die Herstellung von Polymethylenharnstoff ist seit langem bekannt und kann beispielsweise durch säurekatalysierte Gelierung einer Harnstoff-Formaldehyd-Lösung bzw. eines wasserverdünnbaren Harnstoff-Formaldehyd-Konzentrats erfolgen, wie beispielsweise beschrieben in Renner, Makromolekulare Chemie 149, 1 (1971). Des weiteren ist zum Beispiel in der DE-AS-1907914 die Herstellung von feinteiligen Aminoharzfeststoffen auf der Basis von Harnstoff-Formaldehydkondensaten durch säurekatalysierte Polykondensation in wäßrigem Medium beschrieben.

Durch geeignete Verfahrensführung und/oder anschließendes Granulieren kann ein gewünschtes Teilchengrößenspektrum erhalten werden. Auch die Form der Teilchen kann gesteuert werden, wobei erfindungsgemäß kugelförmige Partikel besonders geeignet sind. Bevorzugte Teilchengrößen, die in den absorbierenden Artikeln gemäß der vorliegenden Erfindung Verwendung finden können, sind kleiner als 2 mm, insbesondere kleiner als 0,8 mm. Bevorzugte Bereiche sind 100 bis 2000 µm (0,1 bis 2 mm), insbesondere 200 bis 800 µm (0,2 bis 0,8 mm).

Bei der Verwendung von PMH-Polymeren als rieselfähigen Stoffen in den erfindungsgemäßen absorbierenden Artikeln ist es wichtig, daß während des Gebrauchs der Artikel keine gesundheitsschädlichen Substanzen entstehen können. Bei der vorstehend genannten, säurekatalysierten Polykondensation von Formaldehyd und Harnstoff in wäßrigem Medium zu Polymethylenharnstoff können ethergruppenhaltige Nebenprodukte entstehen. Testet man ein handelsüblich erhältliches PMH-Material daher auf den Gehalt an Formaldehyd, so können diese ethergruppenhaltigen Nebenprodukte gespalten werden und zu einer positiven Reaktion bei einem Test auf Formaldehyd führen. Der Reaktionsablauf läßt sich formelmäßig in etwa wie folgt darstellen (nach Saechtling, Kunststoff-Taschenbuch, 26. Auflage, Carl Hanser Verlag, München, Wien (1995)).

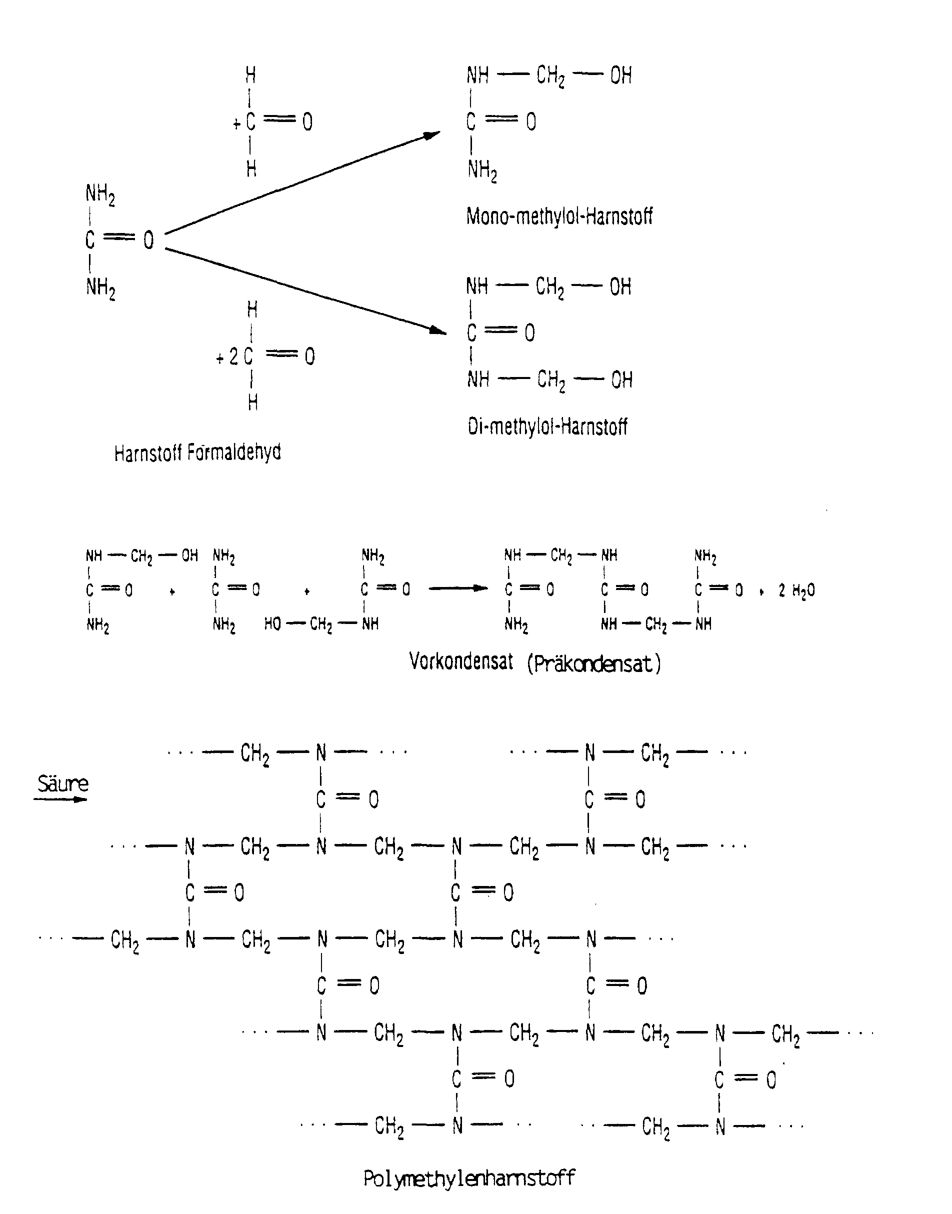

Wie aus dem vorstehenden Formelschema ersichtlich ist, 5 entsteht bei der Umsetzung von Harnstoff und Formaldehyd in Abhängigkeit von dem stöchiometrischen Verhältnis der Reaktanden entweder Monomethylolharnstoff (Verhältnis Harnstoff : Formaldehyd 1 : 1) oder Dimethylolharnstoff (Verhältnis Harnstoff : Formaldehyd 1 : 2). Bei zwischen dem Verhältnis 1 : 1 und 1 : 2 liegenden Verhältnissen werden beide Reaktionsprodukte (Monomethylolharnstoff, Dimethylolharnstoff) anteilmäßig gebildet. Die Reaktionsprodukte setzen sich unter den gewählten Reaktionsbedingungen (basisch, Temperatur zwischen 50 und 100°C) unter Wasserabspaltung zu einem Vorkondensat (Präkondensat) um. Das Vorkondensat wird anschließend mittels Säurekatalyse zu Polymethylenharnstoff vernetzt.

Bei den vorstehend beschriebenen Verfahren entsteht neben dem reinem Polymethylenharnstoff auch ein kleinerer Anteil an PMH, welcher Ethergruppen aufweist, dargestellt im nachfolgenden Formelschema:

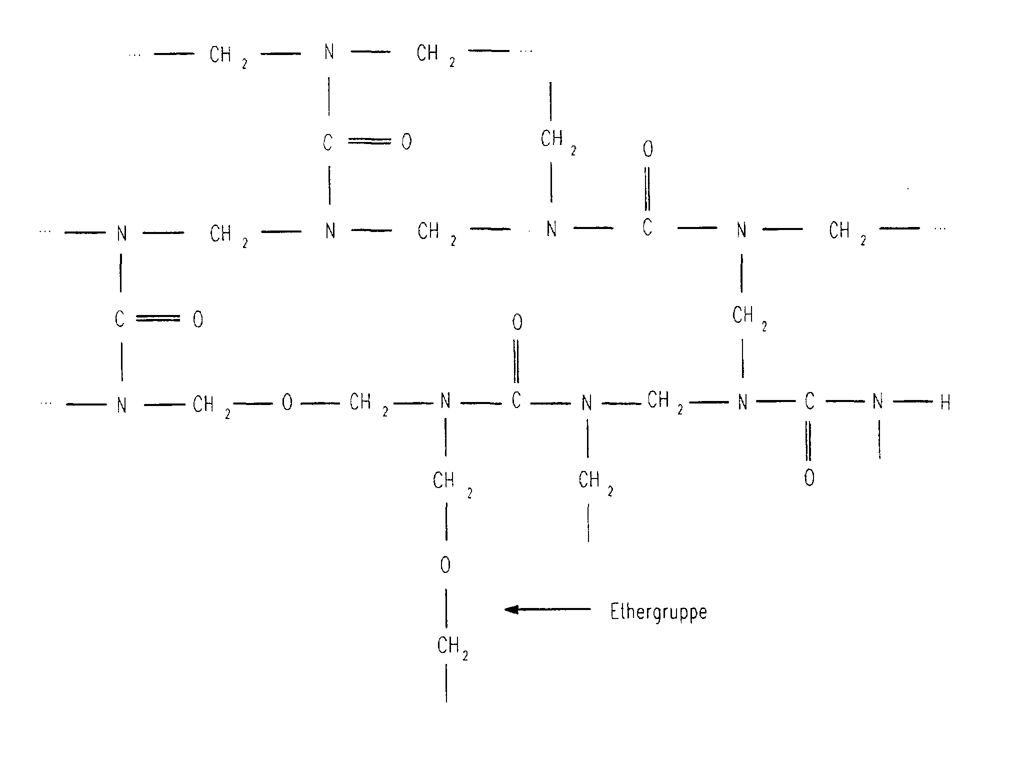

Für den Einsatz in den erfindungsgemäßen absorbierenden Artikeln ist es vorteilhaft, Material zu verwenden, welches möglichst wenig, im Idealfall überhaupt keine, Etherbrücken aufweist.

Es ist somit erfindungsgemäß besonders bevorzugt, ein ethergruppenfreies PMH-Material als rieselfähig verbleibendes Saug- und Speichermaterial in dem absorbierenden Artikel einzusetzen. Ein herkömmliches Syntheseverfahren, bei dem Harnstoff und Formaldehyd in einer Additionsreaktion zu einem Vorkondensat (Präkondensat) umgesetzt werden und bei dem anschließend durch säurekatalysierte Polykondensation das Polymethylenharnstoff-Material hergestellt wird, wird erfindungsgemäß derart abgewandelt, daß nach dem Polykondensationsschritt das ausgefällte Material mit einer Säure, vorzugsweise im pH-Bereich zwischen 1 und 2, gewaschen wird. Erst im Anschluß an den zusätzlichen Säurewaschschritt kann dann eine Behandlung mit sog. "Formaldehydfängern" erfolgen. Dadurch kann ein ethergruppenfreies bzw. formaldehydfreies PMH-Material erhalten werden.

PMH in partikulärer Form kann beispielsweise nach folgendem Verfahren hergestellt werden: eine 30%-ige Formalinlösung, Harnstoff sowie gegebenenfalls Zuschläge (z.B. Schutzkolloide) werden in einem geschlossenen Kessel unter Rühren präkondensiert. Die Temperatur in dem Rührkessel wird zwischen 70 und 90°C und der pH-Wert zwischen 8 und 9 gehalten. Die Herstellung des Präkondensats ist nach etwa 30 bis 90 Minuten beendet. Das Präkondensat wird anschließend mit Säure, z.B. mit Salzsäure, Zitronensäure oder Sulfaminsäure, katalytisch ausgefällt. Der pH-Wert der verwendeten Säure liegt dabei vorzugsweise zwischen 1 und 2. Das ausgefällte Produkt ist Polymethylenharnstoff, der aber noch Ethergruppen enthalten kann. Zur Entfernung dieser Ethergruppen wird der Polymethylenharnstoff nochmals mit einer Säure, beispielsweise den vorgenannten Säuren Salzsäure, Zitronensäure oder Sulfaminsäure, bei pH 1 bis 2 gewaschen. Anschließend wird das PMH-Präzipitat mit einer neutralen Flüssigkeit gewaschen und dann mit sog. Formaldehydfängern, wie z.B. Natriumsulfit, Triethanolamin oder einem Harnstoff-Formaldehyd-Copolymer behandelt. Daran schließt sich wieder eine Wäsche und eine Trocknung des erhaltenen Materials bei beispielsweise 100 bis 110°C an. Daran kann sich eine weitere Nachbehandlung, wie z.B. eine gezielte Granulierung, anschließen. Die Granulierung kann mittels des Einsatzes natürlicher Substanzen, wie z.B. Cellulose, Stärke oder deren Derivate, unterstützt werden.

Das so gewonnene, granulierte PMH-Material ist ethergruppenfrei und somit hervorragend zum Einsatz in absorbierenden Artikeln geeignet, da es sich um ein besonders reines, keine schädlichen bzw. hautirritierenden Stoffe enthaltendes und abgebendes Material handelt.

Neben dem PMH-Material können in dem Saugkörper des absorbierenden Artikels auch noch andere Materialien enthalten sein. Die Materialzusammensetzung kann dabei derart gewählt werden, daß die vorgenannten Funktionen von einem Material übernommen oder auf unterschiedliche Materialien verteilt werden. Diese Materialien können sein: Superabsorber, superabsorbierendes Material in Partikelform, superabsorbierende Fasern, Zeolithe, Fasern von Zellstoff, Zellwolle oder Kunststapelfasern unterschiedlichster Länge, Styropor etc.

Hinsichtlich der Eigenschaften von Polymethylenharnstoffpartikeln ist noch anzumerken, daß diese Partikel eine zeolithartige Struktur aufweisen und damit auch ähnlich wie Zeolithe wirken. Zeolithe werden in Hygieneartikeln üblicherweise zur Geruchsbindung eingesetzt. Wenn als flüssigkeitsaufsaugendes bzw. flüssigkeitsspeicherndes Material PMH verwendet wird, kann auf den Einsatz von Zeolithen zur Geruchsbindung verzichtet werden, was einen weiteren Vorteil des erfindungsgemäßen absorbierenden Artikels darstellt. PMH hat darüberhinaus auch Vorteile, was die Menge an saugfähigem einzusetzenden Material angeht. Verglichen beispielsweise mit der Saugleistung von Zellstoff schneidet PMH mehr als doppelt so gut ab. Auch die Kosten für einen PMH-haltigen Saugkörper liegen erheblich unter den entsprechenden Kosten für einen Zellstoffsaugkörper. Im Vergleich zu Polyacrylaten, welche als Superabsorber Verwendung finden, liegen die Kosten für PMH in der selben Größenordnung wie für Zellstoff.

Im folgenden sind die Meßergebnisse von Adsorptionsuntersuchungen an Polymethylenharnstoff (Chargenbezeichnung P 124), Polymethylenharnstoff/Polyacrylat-Mischungen (Chargenbezeichnung P 124 + AK) und Polyacrylat (Chargenbezeichnung AK) beschrieben. Die in Tabelle 1 aufgeführten Eigenschaften wurden mittels eines Tensiometers K121 der Firma Krüss ermittelt.

**Tabelle 1**

| **Probe** | **δ [grd]** | **vSteig x 10**^{**2**} **[g/s]** | **max. Wasser- aufnahme [g/g]** |
|---|---|---|---|
| P124 | 56,8 | 3,266 | 16,1 |
| P124+3%AK | 66,1 | 2,42 | 20,3 |
| P124+6%AK | 83,1 | 0,715 | 15,5 |
| AK | 76,9 | 1,346 | 1,6 |

In der zweiten Spalte von Tabelle 1 ist der jeweilige Benetzungswinkel δ des untersuchten Materials angegeben. Die dritte Spalte in Tabelle 1 gibt die Steiggeschwindigkeit des Materials an, wobei die Steighöhe in Form einer Gewichtszunahme des Materials ermittelt wurde.

Wie aus Tabelle 1, vierte Spalte, des weiteren ersichtlich ist, weist 100% P124 ein maximales Wasseraufnahmevermögen von 16,1 g/g Material auf. Eine Mischung aus 97% P124 und 3% AK steigert das maximale Wasseraufnahmevermögen auf 20,3 g/g, und beim Ersatz weiterer 3% P124 durch AK fällt der Wert wieder auf 15,5 g/g ab. Alle vorstehend angegebenen Werte zeigen, daß die entsprechenden Materialien für den Einsatz in Saugkörpern von absorbierenden Artikeln geeignet sind.

Das maximale Wasseraufnahmevermögen der jeweiligen Materialien wurde unter Bedingungen bestimmt, bei denen für die Materialien keine Volumenvergrößerung möglich war, d.h. ein Quellen unterbunden wurde. Dies erklärt das äußerst geringe Wasseraufnahmevermögen von 1,6 g/g des Polyacrylatmaterials.

Der Polymethylenharnstoff P124 behält seine Rieselfähigkeit bis zu einer Wasseraufnahme von 10,5 g/g bei. Erst wenn ein Wert von 10,5 g/g überschritten wird, beginnt das Material leicht klumpig zu werden, und zwar bis ein Wert von 12,8 g/g erreicht ist. Bei einer über 12,8 g/g hinausgehenden Wasserzufuhr tritt eine krümelige Materialstruktur auf, die bei 18,8 g/g in eine teigige, fließende Konsistenz übergeht. Im Gegensatz dazu konnte eine Rieselfähigkeit von reinem Polyacrylat (AK) nicht bestimmt werden, da dieses Material bereits bei geringster Wasseraufnahme gelierte und sich klebrig miteinander verband bzw. an der Gefäßwand haften blieb.

Ein leichtes Quellen des Materials und Ansätze von Gelblocking-Effekten konnten bei PMH/Polyacrylat-Mischungen im Verhältnis 95:5 beobachtet werden. Je höher der Anteil von Polyacrylat in der Mischung wurde, desto stärkeres Quellen und Gelblocking wurde beobachtet. Bei reinem Polymethylharnstoff P124 wurde auch bei starker Wasseraufnahme kein Quellen, d.h. keine Volumenzunahme, festgestellt. Schließlich wurden auch noch maximal mit Wasser beladene PMH-und Polyacrylat-Materialien mit Druck belastet. Während es nicht möglich war, aus PMH Wasser auszudrücken, gab Polyacrylat bei stärkerer Druckbelastung Wasser ab.

Ein weiterer Aspekt der Erfindung befaßt sich mit der verbesserten Verhinderung des Entstehens unangenehmer Gerüche beim Gebrauch von absorbierenden Artikeln. Erfindungsgemäß wird dazu vorgeschlagen, auf oder in dem saugfähigen Material, welches auch nach Beaufschlagung mit Flüssigkeit rieselfähig bleibt, adsorptiv gebunden oder immobilisiert bakterizide, fungizide und/oder viruzide Substanzen aufzubringen. Wird als saugfähiges Material beispielsweise PMH verwendet, so weist dieses die oben beschriebene zeolithartige Struktur auf, d.h. das Material hat neben seiner äußeren Oberfläche auch noch eine große innere Oberfläche, die zwischen 10 und 700 m²/g betragen kann. Dabei ist es wichtig, daß die bakteriziden, fungiziden und viruziden Substanzen auf dem saugfähigen Material immobilisiert werden, da eine Freisetzung der Substanzen zu Hautirritationen der Träger der absorbierenden Artikel führen könnte. Für die unangenehmen Gerüche beim Gebrauch von absorbierenden Artikeln sind vor allem Stoffwechselprodukte von Mikroorganismen verantwortlich, d.h. die Geruchsbildung kann wirksam unterdrückt oder unterbunden werden, wenn das Wachstum und/oder die Vermehrung der Mikroorganismen gehemmt wird bzw. diese abgetötet werden können. Mit den genannten Substanzen ist eine Hemmung und eine Unterdrückung des Wachstums der Mikroorganismen möglich, so daß zusätzlich zur schon guten Geruchsbildungsverhinderung des PMH-Materials an sich diese Geruchsbildung durch die zusätzliche Anwendung der genannten bakteriziden, fungiziden und viruziden Substanzen weiter verhindert werden kann, was dem Träger eines erfindungsgemäßen absorbierenden Artikels weitere Sicherheit vor unerwünschten Nebenwirkungen gibt.

Geeignete bakterizide Substanzen sind beispielsweise chlorierte Laevulinsäure und Alkyldimethylbenzylammoniumhalogenide.

Neben dem Saugkörper, welcher die auch bei Flüssigkeitsbeaufschlagung rieselfähigen Bestandteile beinhaltet, kann der Saugkörper auch noch andere Bereiche aufweisen. In einem solchen Fall ist der Saugkörperanteil, welcher das rieselfähige Material enthält, vorzugsweise als mindestens ein Kernstück ausgestaltet, dessen Länge l kleiner ist als die Länge L des absorbierenden Artikels und dessen Breite b kleiner ist als die Breite B des absorbierenden Artikels.

Der Saugkörper kann mit der darunterliegenden Schicht über die gesamte Auflagefläche, beispielsweise durch ein Haftmittel, verbunden sein. Es kann jedoch auch ausreichen, nur einen Teil des Saugkörpers mit der darunterliegenden Schicht zu verbinden. Hier sind wieder verschiedenste Ausführungsformen denkbar, beispielsweise eine streifenweise Befestigung, indem der Saugkörper mit Haftmittelstreifen auf der Unterlage fixiert wird. Allgemein ausgedrückt ist die Fläche bzw. der Flächenanteil, über den der Saugkörper mit der darunterliegenden Schicht verbunden ist, kleiner als die Fläche bzw. der Flächenanteil, l x b. Die Länge λ der Verbindung Saugkörper/darunterliegende Schicht ist dabei kleiner oder gleich der Länge l und die Breite β der Verbindung Saugkörper/darunterliegende Schicht ist kleiner oder gleich der Breite b.

Der Saugkörper kann aus einer Kammer bestehen oder unterteilt sein in mehrere Unterkammern, die vollständig voneinander getrennt sein oder miteinander kommunizieren können, wobei dann bei Druckbelastung auf die Kammer die Partikel auch in eine benachbarte Kammer ausweichen können.

Ist der Saugkörper und/oder das Kernstück des Saugkörpers in mehrere Kammern unterteilt, so können die vorhandenen Trennwände in Längs- und/oder in Querrichtung zu dem absorbierenden Artikel verlaufen. Durch eine Längs- oder Querwand erfolgt zum Beispiel eine Unterteilung in zwei Unterkammern. Zwei Längswände ergäben eine dreikammerige Anordnung, und kommt zu den beiden Längswänden noch eine Querwand hinzu, so ist der Saugkörper oder das Kernstück davon in sechs Kammern unterteilt.

Eine Kammer kann dabei jeweils zu 100% mit saugfähigem Material gefüllt sein. Es hat sich jedoch auch als günstig herausgestellt, wenn nicht die gesamte Kammer, welche das auch bei Flüssigkeitsbeaufschlagung noch rieselfähig verbleibende Material enthält, mit Material gefüllt ist. So können zum Beispiel 50 bis 100%, vorzugsweise 60 bis 90% und insbesondere 80% einer Kammer mit saugfähigem Material angefüllt sein. Ist der Saugkörper mehrkammerig ausgestaltet, so gelten für einzelne Kammern entsprechende bevorzugte Füllmengen. Wenn weniger als 60% einer Kammer gefüllt sind, so ist es des weiteren vorteilhaft, wenn innerhalb einzelner Kammern noch sogenannte "Rieselbarrieren" angeordnet sind, welche verhindern, daß alles saugfähige Material sich in einer Ecke der Kammer ansammelt. Auch können, wie vorstehend bereits angedeutet, die Trennwände zwischen einzelnen Kammern kleine Öffnungen aufweisen, so daß auch ein begrenzter Materialaustausch zwischen einzelnen Kammern erfolgen kann, d.h. daß einzelne Kammern miteinander kommunizieren können. Eine nicht vollständige Füllung einer einzelnen Kammer ist insbesondere immer dann notwendig, wenn die Kammer quellfähiges Material, wie beispielsweise einen Superabsorber enthält.

Gemäß eines weiteren Aspekts der vorliegenden Erfindung können bei Vorliegen von mehreren, den Saugkörper bildenden Kammern diese auch jeweils mit unterschiedlichen Materialien gefüllt sein.

So kann beispielsweise bei einer Saugkörperausgestaltung mit drei Kammern, wobei die Trennwände längs oder quer zum absorbierenden Artikel verlaufen können, die zentrale Kammer mit PMH oder PMH/Superabsorber-Mischungen gefüllt sein, während die (bei Längstrennwänden) lateral gelegenen Kammern bzw. die (bei Quertrennwänden) am Vorder- und Hinterende gelegenen Kammern mit Superabsorbern gefüllt sein können.

Wenn Mischungen aus beispielsweise PMH und Superabsorbern verwendet werden, ist darauf zu achten, daß sich diese Mischungen nicht entmischen, da dies dazu führen könnte, daß nicht alles potentiell saug- bzw. speicherfähige Material nutzbar ist, d.h. sog. "totes Material" entstehen könnte.

Die vorliegende Erfindung stellt somit sich den Körperkonturen bestens anpassende absorbierende Artikel zur Verfügung. Die Artikel sind des weiteren dadurch ausgezeichnet, daß sie bei Beaufschlagung geeigneter Saugkörpermaterialien mit Flüssigkeit keine Volumenvergrößerung erfahren, d.h. nicht "quellen". Schließlich können die erfindungsgemäßen Artikel auch im deformierten Zustand optimal Flüssigkeiten aufnehmen.

Auch passen sich die erfindungsgemäßen absorbierenden Artikel optimal den Körperkonturen des Trägers an. Die Artikel können somit sehr nahe am Körper getragen werden (anatomisches "Form-Fitting"), was den Vorteil hat, daß eine Flüssigkeit sofort nach dem Austreten aus dem Körper aufgenommen werden kann, wodurch sich beim Träger kein Nässegefühl auf der Haut ausbildet. Das Trockenheitsgefühl auf der Haut des Trägers wird auch dadurch erreicht, daß das saugfähige Material gezielt zentriert in den absorbierenden Artikeln gemäß der Erfindung angeordnet ist. Schließlich erlaubt die erfindungsgemäße Ausgestaltung der absorbierenden Artikel auch noch, daß im Fall einer Damenbinde deren Enden extrem dünn gehalten werden können, was ein sehr diskretes Tragen eines entsprechenden Artikels ermöglicht.

Neben dem vorstehend als Saugkörper bezeichneten, gleichzeitig als Flüssigkeitsspeicherschicht dienenden Element (= Primärspeicher) kann der erfindungsgemäße absorbierende Artikel auch noch eine weitere Speicherschicht (sog. Sekundärspeicher) aufweisen. Diese weitere Speicherschicht ist vorzugsweise als Bahn zwischen dem Saugkörper und der flüssigkeitsundurchlässigen, dem Körper abgewandten Schicht ausgebildet. Dabei kann dieses die weitere Speicherschicht bildende Material auch saugend wirken, um eine bessere Flüssigkeitsverteilung zu bewirken. Diese weitere Speicherschicht ist nur für "Notfälle" gedacht, wenn der Saugkörper (Primärspeicher) aus irgendwelchen Gründen seine Kapazitätsgrenze überschreiten sollte. Geeignete Materialien für die weitere Speicherschicht (Sekundärspeicher) sind beispielsweise Coform-Materialien, Zellstoff, Zellstofffaser-Mischungen (Air-laid), Vliese oder Tissuewatte.

Die erfindungsgemäßen absorbierenden Artikel mit dem neuartigen Saugkörper können z.B. im Bereich der Hygiene, wie der Damenhygiene, beispielsweise als Damenbinde, insbesondere ultradünne Damenbinde, oder Slipeinlage Verwendung finden. Daneben kann der erfindungsgemäße absorbierende Artikel beispielsweise aber auch als Kinder-Wegwerfwindel oder Inkontinenzeinlage ausgestaltet sein.

Wenn der absorbierende Artikel ein Artikel gemäß des ersten Aspekts der Erfindung ist, d.h. eine bei Verwendung des Artikels dem Körper zugewandte, flüssigkeitsdurchlässige Abdeckschicht aufweist, so kann unterhalb dieser flüssigkeitsdurchlässigen Schicht eine weitere Deckschicht angeordnet sein, welche eine zentrale, über dem Saugkörper gelegene Öffnung (sog. "Port-Hole") aufweist. Entsprechende absorbierende Artikel sind beispielsweise in der deutschen Patentanmeldung Nr. 19640451.7 beschrieben. Dieses Port-Hole-Design ist insbesondere für Damenhygieneartikel vorteilhaft.

Der in der genannten deutschen Patentanmeldung angegebene absorbierende Artikel kann folgenden Aufbau aufweisen. Auf der beim Gebrauch des absorbierenden Artikels dem Körper abgewandten Seite befindet sich eine flüssigkeitsundurchlässige Schicht. Oberhalb dieser flüssigkeitsundurchlässigen Schicht ist eine Primärspeicherschicht angeordnet. Daraufhin schließt sich nach oben eine Sekundärspeicherschicht an. Oberhalb der Sekundärspeicherschicht ist eine Kompensationsschicht angeordnet, und oberhalb der Kompensationsschicht befindet sich eine Deckschicht, welche eine zentrale Öffnung aufweist. Eine entsprechende, mit Öffnung versehene Deckschicht kann auch in den absorbierenden Artikeln gemäß der vorliegenden Erfindung vorhanden sein. Schließlich enthält der absorbierende Artikel gemäß der vorliegenden Erfindung noch eine obere, bei Gebrauch des absorbierenden Artikels dem Körper zugewandte flüssigkeitsdurchlässige Schicht. Die Sekundärspeicherschicht kann zumindest einen verdichteten Bereich aufweisen.

Als Material für die Sekundärspeicherschicht ist beispielsweise Zellstoff geeignet. Lokale Verdichtungen in dem Sekundärspeicher können beispielsweise durch Einprägen von Rillen in den Speicher erfolgen. Das unterhalb der eingeprägten Rillen gelegene Speichermaterial ist dabei verdichtet, während die Rillen zur gerichteten Flüssigkeitsverteilung auf der Speicherschicht bzw. in dem absorbierenden Artikel beitragen.

Die Deckschicht, welche die zentrale Öffnung aufweist, wird z.B. aus einer Mischung aus Zellstoff und polymerisiertem Alken hergestellt. Entsprechende Mischungen enthalten günstigerweise mindestens 50 Gew.-% polymerisiertes Alken. Sehr gute Ergebnisse werden erzielt, wenn der Anteil an polymerisiertem Alken 50-80 Gew.-%, insbesondere 60 Gew.-% beträgt. Die Deckschicht kann auch aus zwei Schichten aufgebaut sein, derart, daß eine erste Schicht aus einem Gemisch aus Zellstoff und polymerisiertem Alken auf einer zweiten Trägerschicht aus polymerisiertem Alken aufgebracht ist, wobei die erste Schicht aus einem Gemisch aus Zellstoff und polymerisiertem Alken mit der bei Gebrauch des absorbierenden Artikels dem Körper zugewandten flüssigkeitsundurchlässigen Schicht und die zweite Trägerschicht mit der Kompensationsschicht in Verbindung steht. Bevorzugte polymerisierte Alkene sind Polyethylen, Polypropylen und Gemische aus Polyethylen und Polypropylen. Die Deckschicht kann des weiteren ein Pigment, wie Titandioxid, enthalten. Das Material der Kompensationsschicht ist vorteilhafterweise aus einem Vliesmaterial aufgebaut. Das Vliesmaterial kann polymerisiertes Alken und /oder Bikomponentenfasern enthalten. Auch kann die Kompensationsschicht auf der der Speicherschicht zugewandten Oberfläche mit einer oberflächenaktiven Substanz, welche beispielsweise siliconhaltig sein kann, beschichtet sein. Die Primärspeicherschicht kann beispielsweise aus einem UCTAD-Material (uncreped through air dried -Material), Tissuewatte oder einem polymeren Alken bestehen. Die Primärspeicherschicht ist vorteilhafterweise so aufgebaut, daß deren Randbereiche derart eingefaltet sind, daß diese sich gegenseitig überlappen.

Sowohl die flüssigkeitsundurchlässige Schicht als auch die flüssigkeitsdurchlässige Schicht können aus einem polymerisierten Alken, wie beispielsweise Polyethylen, Polypropylen oder einem Gemisch daraus, aufgebaut sein. Zur Befestigung des erfindungsgemäßen absorbierenden Artikels an einem Kleidungsstück kann an der flüssigkeitsundurchlässigen Schicht mindestens ein Haftelement und/oder eine Haftschicht angebracht sein. Des weiteren kann der erfindungsgemäße absorbierende Artikel auch seitlich angeordnete Flügel aufweisen.

Die Erfindung wird nachfolgend anhand der Zeichnungen näher erläutert. Es zeigen:
- Fig. 1: eine perspektivische Ansicht eines erfindungsgemäßen absorbierenden Artikels in Form einer Damenbinde;
- Fig. 2: eine perspektivische Ansicht eines erfindungsgemäßen absorbierenden Artikels in Form einer Damenbinde, teilweise im Anschnitt;
- Fig. 3: einen Querschnitt durch eine Ausführungsform des absorbierenden Artikels gemäß Fig. 1 entlang der Linie A-A von Fig. 1;
- Fig. 4: einen Querschnitt durch eine weitere Ausführungsform eines erfindungsgemäßen absorbierenden Artikels in Form einer Damenbinde;
- Fig. 5: einen Längsschnitt durch eine Ausführungsform des absorbierenden Artikels gemäß Fig. 1 entlang der Linie B-B von Fig. 1;
- Fig. 6: in schematischer Form die Längen- und Breitenverhältnisse eines erfindungsgemäßen absorbierenden Artikels und eines dazugehörigen Kernstücks eines Saugelements;
- Fig. 7a-z: in Draufsicht Formen, welche der Saugkörper bzw. das Kernstück des Saugkörpers eines erfindungsgemäßen absorbierenden Artikels einnehmen können;
- Fig. 8a-c: Kammerungen (in Längsrichtung) des Saugkörpers bzw. Kernstück des Saugkörpers eines erfindungsgemäßen absorbierenden Artikels;
- Fig. 8d-f: Kammerungen (in Querrichtung) des Saugkörpers bzw. Kernstück des Saugkörpers eines erfindungsgemäßen absorbierenden Artikels;
- Fig. 9: Kammerungen (in Längs-und Querrichtung) des Saugkörpers bzw. Kernstücks des Saugkörpers eines erfindungsgemäßen Artikels;
- Fig. 10a-d: Verformungsmöglichkeiten des Saugkörpers bzw. Kernstücks des Saugkörpers eines erfindungsgemäßen Artikels;
- Fig. 11a: das Drehmoment eines Polymethylenharnstoffpulvers (PMH) während der Benetzung mit steigender Menge Blutersatzlösung (BEL);
- Fig. 11b: das Drehmoment einer Mischung aus 8 Masseteilen PMH und 1 Masseteil Polyacrylat (SAP) während der Benetzung mit steigender Menge BEL;
- Fig. 11c: das Drehmoment einer Mischung aus 4 Masseteilen PMH und 1 Masseteil SAP während der Benetzung mit steigender Menge BEL;
- Fig. 11d: das Drehmoment einer Mischung aus 2 Masseteilen PMH und einem Masseteil SAP während der Benetzung mit steigender Menge BEL;
- Fig. 11e: das Drehmoment einer Mischung aus gleichen Masseteilen PMH und SAP während der Benetzung mit steigender Menge BEL;
- Fig. 11f: das Drehmoment einer Mischung aus 1 Masseteil PMH und 2 Masseteilen SAP während der Benetzung mit steigender Menge BEL;
- Fig. 12: ein Diagramm zur Darstellung der Maxima der Drehmomente von PMH bzw. verschiedener PMH/SMH-Mischungen bei Benetzung mit BEL;
- Fig. 13: eine perspektivische Ansicht eines weiteren erfindungsgemäßen absorbierenden Artikels in Form einer Damenbinde;
- Fig. 14: einen Querschnitt durch den absorbierenden Artikel gemäß Fig. 13 entlang der Linie IV-IV;
- Fig. 15: eine perspektivische Ansicht eines weiteren erfindungsgemäßen absorbierenden Artikels in Form einer Damenbinde;
- Fig. 16: einen Querschnitt durch den absorbierenden Artikel gemäß Fig. 15 entlang der Linie VI-VI;
- Fig. 17: eine perspektivische Ansicht eines weiteren erfindungsgemäßen absorbierenden Artikels in Form einer Damenbinde;
- Fig. 18: einen Querschnitt durch den absorbierenden Artikel gemäß Fig. 17 entlang der Linie II-II.
- Fig. 19: eine perspektivische Ansicht einer weiteren Ausführungsform des erfindungsgemäßen absorbierenden Artikels in Form einer Damenbinde; und
- Fig. 20: einen Querschnitt durch den absorbierenden Artikel gemäß Fig. 19 entlang der Linie XX-XX.

Obwohl die erfindungsgemäßen absorbierenden Artikel nachfolgend anhand von Damenbinden im Detail gezeigt werden, ist klar, daß sich die vorliegende Erfindung nicht auf Damenbinden beschränkt, sondern alle absorbierenden Hygieneartikel umfaßt.

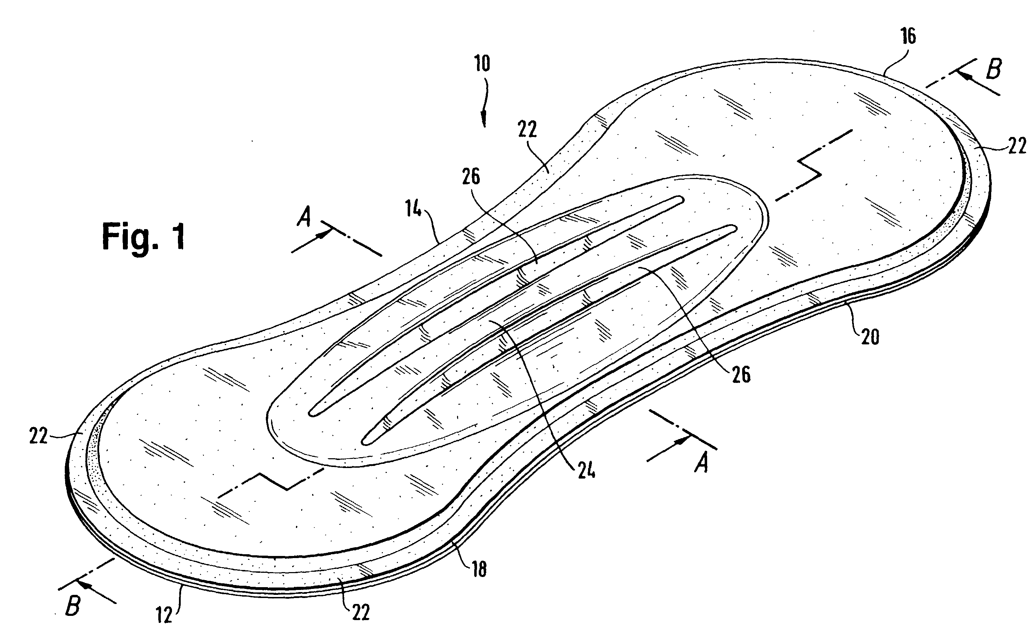

Fig. 1 zeigt eine erfindungsgemäße Damenbinde 10, welche einen vorderen Bereich 12, einen mittleren Bereich 14 und einen Endbereich 16 aufweist. Die beim Tragen der Damenbinde 10 dem Körper der Trägerin zugewandte flüssigkeitsdurchlässige Schicht 18 und die dem Körper abgewandte flüssigkeitsundurchlässige Schicht 20 sind im Randbereich 22 der Damenbinde 10 miteinander verbunden. Zentral erstreckt sich in Längsrichtung der Damenbinde 10 der nicht sichtbare Saugkörper, welcher bewirkt, daß die flüssigkeitsdurchlässige Schicht 18 im Zentralbereich der Damenbinde gegenüber dem vorderen Bereich 12 und dem Endbereich 16 erhoben ist. Weiterhin erkennbar sind zwei Längsrillen 26 im Zentralbereich 24, welche einerseits die Kammerung des Saugkörpers reflektieren und andererseits zur gerichteten Flüssigkeitsverteilung bei Beaufschlagung der Damenbinde dienen.

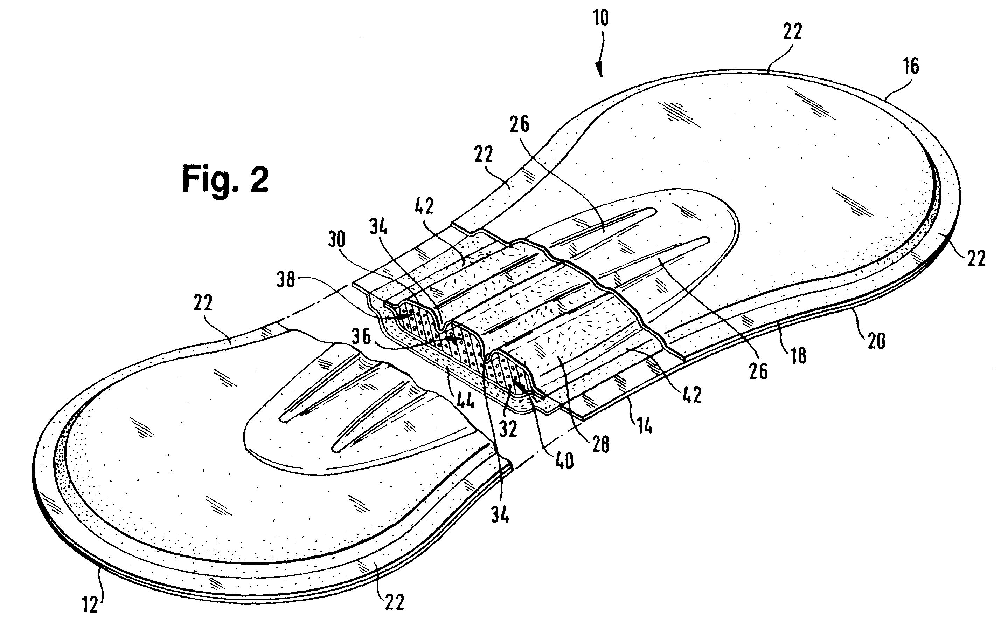

In Fig. 2 ist ein erfindungsgemäßer absorbierender Artikel 10 perspektivisch teilweise im Anschnitt gezeigt. Man erkennt wiederum den vorderen Bereich 12, den mittleren Bereich 14 und den Endbereich 16 des Artikels. Die flüssigkeitsdurchlässige Schicht 18 und die flüssigkeitsundurchlässige Schicht 20 (Wäscheschutzfolie) sind im Randbereich 22 miteinander verbunden. Das Kernstück 28 des Saugkörpers des erfindungsgemäßen Artikels ist im Zentralbereich desselben angeordnet und erstreckt sich in Längsrichtung des Artikels. Das Kernstück umfaßt eine (flüssigkeitsdurchlässige) Umhüllung 30 aus einem Vliesmaterial. In der Umhüllung eingeschlossen ist das auch nach Beaufschlagung mit einer Flüssigkeit noch rieselfähig verbleibende Material 32, im vorliegenden Fall Polymethylenharnstoff mit einer Teilchengröße zwischen 200 und 800µm, wobei die einzelnen Teilchen weitgehend Kugelform aufweisen. Bei der in Fig. 2 gezeigten Ausführungsform ist die Umhüllung 30 nahezu vollständig mit Material 32 gefüllt, was keine Probleme bereitet, da dieses Material auch bei Flüssigkeitsbeaufschlagung nicht quillt und die Gefahr, daß die Umhüllung 30 platzt, somit nicht gegeben ist.

In Längsrichtung des absorbierenden Artikels 10 erstrecken sich die Längsrillen 26. Die Umhüllung 30 des Kernstücks weist Einschnürungen 34 auf, welche eine gewisse Kompartimentierung des saugfähigen Materials 32 bewirken. Das Kernstück ist dabei in eine zentrale Kammer 36 und seitliche Kammern 38, 40 untergliedert. Wie aus Fig. 2 gut ersichtlich ist, reichen die Abgrenzungswände der einzelnen Kammern nicht bis zur Basis der Umhüllung 30, so daß ein begrenzter Materialaustausch zwischen einzelnen Kammern möglich ist. Die Umhüllung 30 ist bei dem in Fig. 2 gezeigten Ausführungsbeispiel aus zwei Teilen gefertigt, welche im Randbereich 42 miteinander verbunden sind. Durch diese Konstruktion wird die Befüllung des Kernstücks mit absorbierendem Material erleichtert.

Das oval ausgebildete Kernstück 28 des Saugkörpers ist bei der in Fig. 2 gezeigten Ausführungsform des erfindungsgemäßen Artikels des weiteren mit einem saugfähigen, zellstoffhaltigen Material 44 unterlegt. Dieses zellstoffhaltige Material dient einerseits dem Tragekomfort und andererseits als Reservespeicher (Sekundärspeicher) für den Fall, daß die Speicherkapazität des mit dem auch bei Flüssigkeitsbeaufschlagung rieselfähig bleibenden Materials gefüllten Kernstücks überschritten wird. Üblicherweise wird dieser Reservespeicher aber nicht in Anspruch genommen werden müssen, da Untersuchungen ergeben haben, daß z.B. die überwiegende Mehrzahl aller Damenbinden mit weniger als 5 ml Flüssigkeit beaufschlagt wird und dafür die Speicherkapazität des Kernstücks in in jedem Falle ausreichend ist.

Bei Beaufschlagung des erfindungsgemäßen Artikels 10 mit Blut wird dieses zunächst unter Mithilfe der Längsrillen 26 verteilt. Das Blut durchdringt dann die flüssigkeitsdurchlässige Schicht 18 und dringt durch die Umhüllung 30 in das Kernstück 28 mit dem Material 32 ein und wird dort festgehalten.

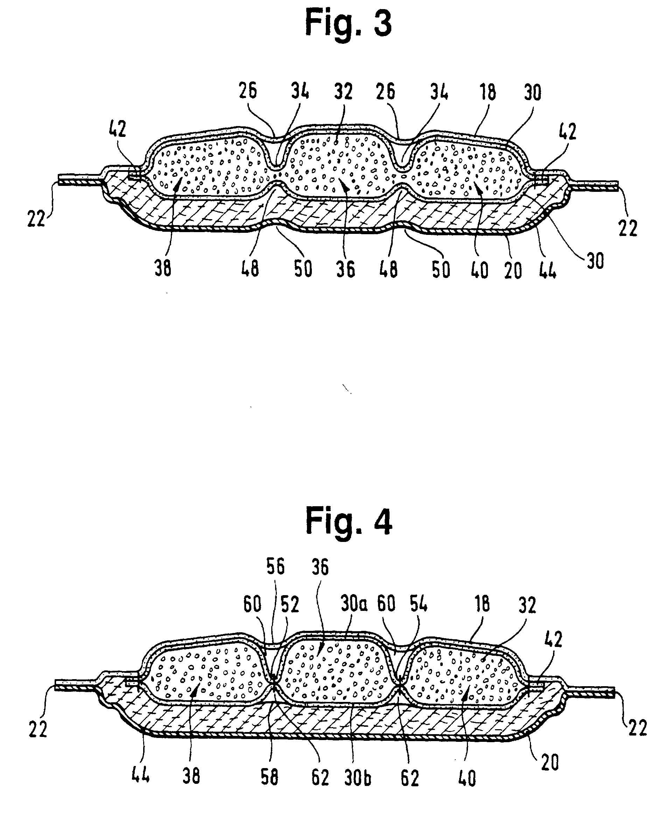

Fig. 3 zeigt einen Querschnitt durch den erfindungsgemäßen absorbierenden Artikel entlang der Linie A-A von Fig. 1. Von oben nach unten gesehen findet man unter der flüssigkeitsdurchlässigen Schicht 18 das Kernstück 28 des Saugkörpers. Dieses ist mit der Umhüllung 30 umschlossen und mit dem auch nach Flüssigkeitsbeaufschlagung rieselfähig bleibenden Material 32 gefüllt. Unterhalb des Kernstücks 32 ist eine Sekundärspeicherschicht aus Zellstoffmaterial 44 angeordnet (dient in erster Linie als Reserve- oder Sekundärspeicher), und der absorbierende Artikel wird nach unten durch die flüssigkeitsundurchlässige Schicht 20, welche aus Polyethylen besteht, abgeschlossen. Eine weitere Besonderheit der in Fig. 3 gezeigten Ausführungsform ist, daß die Umhüllung 30 auch von unten her Einschnürungen 48 aufweist, welche mit den oberen Einschnürungen 34 fluchten. Dadurch ergibt sich eine noch ausgeprägtere Kammerung des Kernstücks des Saugkörpers, wobei ein Materialaustausch zwischen einzelnen Kammern in geringfügigem Umfang möglich bleibt. In dem Randbereich 22 sind die flüssigkeitsdurchlässige Schicht 18 und die flüssigkeitsundurchlässige Schicht 20 miteinander verbunden. Die Verbindung ist im vorliegenden Fall durch Verklebung der Schichten mittels eines Haftmittels erzielt worden. Es ist jedoch auch möglich, die Schichten auf andere Weise miteinander zu verbinden, beispielsweise durch Ultraschall oder Heißsiegeln. In ähnlicher Weise wie der Randbereich 22 sind auch im Randbereich 42 der Umhüllung 30 zwei Schichten miteinander verklebt.

Fig. 4 zeigt einen Querschnitt durch ein weiteres Ausführungsbeispiel eines erfindungsgemäßen absorbierenden Artikels. Gleiche Elemente sind in den Figuren 3 und 4 mit den gleichen Bezugsziffern gekennzeichnet. Bei der Ausführungsform gemäß Fig. 4 sind die seitlichen Kammern 38, 40 von der zentralen Kammer 36 vollständig abgeschlossen, so daß ein Austausch des Materials 32 zwischen den Kammern nicht möglich ist. Die Kammerung erfolgt, indem der obere Bereich 30a der Umhüllung 30 und der untere Bereich 30b der Umhüllung 30 an den Punkten 52, 54 miteinander in Verbindung stehen. Eine dauerhafte Verbindung wird mittels Vernähen der oberen bzw. unteren Umhüllungsschicht erreicht. Die Abkammerung könnte z.B. auch durch Verkleben des oberen Bereichs 30a mit dem unteren Bereich 30b erfolgen. Eine weitere Besonderheit des in Fig. 3 und 4 gezeigten Ausführungsbeispiels ist, daß obere Hohlräume 60 vorhanden sind. In diesen Hohlräumen kann sich die eingedrungene Flüssigkeit sehr gut in Längsrichtung verteilen, was eine gleichmäßige Speicherung derselben über den gesamten Saugkörper bewirkt. Durch diese Anordnung kann die Saug- und Speicherkapazität des Kernstücks optimal genutzt werden.

Mit der Bezugsziffer 58 ist in Fig. 4 die Oberkante der Sekundärspeicherschicht 44 angegeben. Wenn keine Einbuchtungen der flüssigkeitsundurchlässigen Schicht 20 vorhanden sind, können sich kleine untere Hohlräume 62 zwischen einzelnen Kammern des Saugkörpers ausbilden.

Des weiteren kann der Saugkörper des erfindungsgemäßen absorbierenden Artikels auch eine Unterkammerung dergestalt aufweisen, daß es abgetrennte Bereiche gibt (wie aus Fig. 4 ersichtlich, vergl. Punkte 52, 54) neben Bereichen, die einen Materialaustausch zwischen Kammern ermöglichen (wie aus Fig. 3 ersichtlich, Bereiche zwischen den mit den Bezugsziffern 34 und 48 angegebenen Punkten). In Längsrichtung des absorbierenden Artikels gibt es dann unterschiedliche Bereiche zwischen der zentralen Kammer 36 und den seitlichen Kammern 38, 40. Einmal ist Materialaustausch zwischen den Kammern möglich (wenn die Kammern wie in Fig. 3 gezeigt, nicht vollständig voneinander abgetrennt sind), an anderer Stelle ist ein Materialaustausch unterbunden (wie in Fig. 4, Punkte 52, 54 gezeigt).

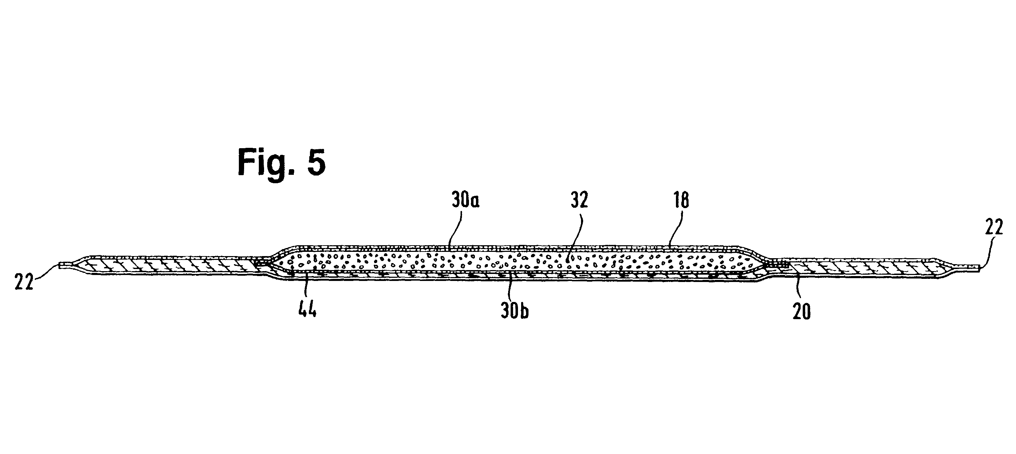

Fig. 5 zeigt einen Längsschnitt durch den absorbierenden Artikel gemäß Fig. 1 entlang der Linie B-B in dieser Figur. Die flüssigkeitsdurchlässige Schicht 18 und die flüssigkeitsundurchlässige Schicht 20 sind im Randbereich 22 miteinander verbunden. Die zentrale Kammer des Kernstücks ist ebenfalls längs angeschnitten und beinhaltet das Material 32.

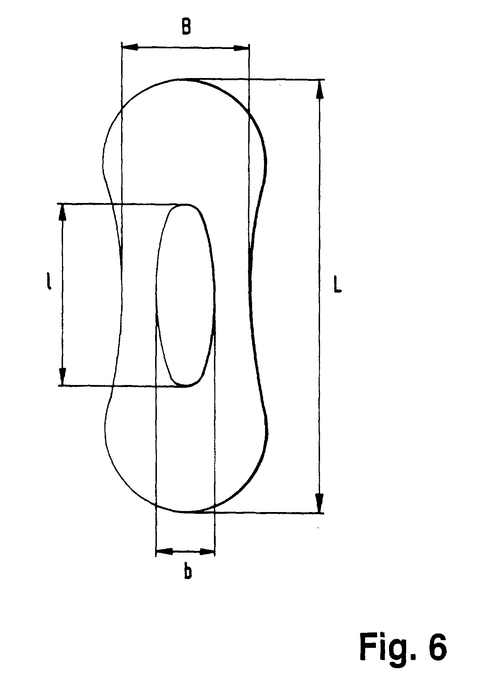

In Fig. 6 ist gezeigt, wie die Abmessungen b und l des Kernstücks des Saugkörpers im Verhältnis zu den Abmessungen B und L des absorbierenden Artikels günstigerweise gestaltet werden können. Es ist in jedem Fall von Vorteil, wenn b kleiner ist als B und l kleiner als L.

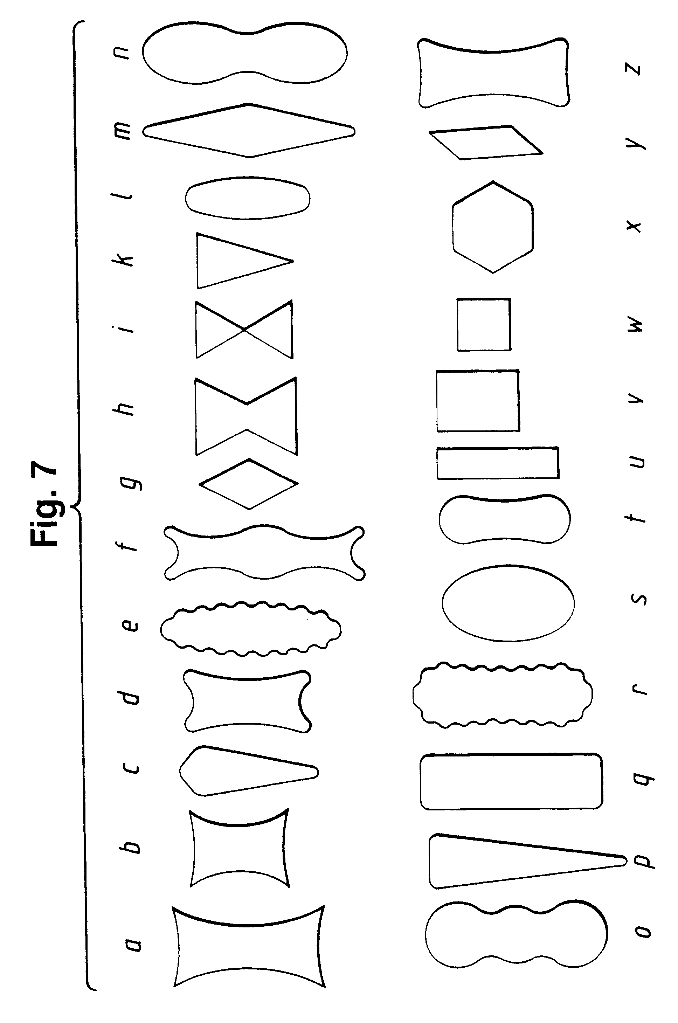

Fig. 7 a bis z zeigt 25 verschiedene Möglichkeiten des Kernstückdesigns. In Abhängigkeit von der Funktion des erfindungsgemäßen absorbierenden Artikels kann ein geeignetes Kernstück ausgewählt werden. Bei einer "Hundeknochen"-Struktur, wie in Fig. 1 gezeigt, bietet es sich beispielsweise an, ein ähnlich geformtes Kernstück zu verwenden (siehe beispielsweise Fig. 6). Aber auch eine ovale Form (Fig. 7b) des Kernstücks kann mit Vorteil eingesetzt werden (vgl. Fig. 1).

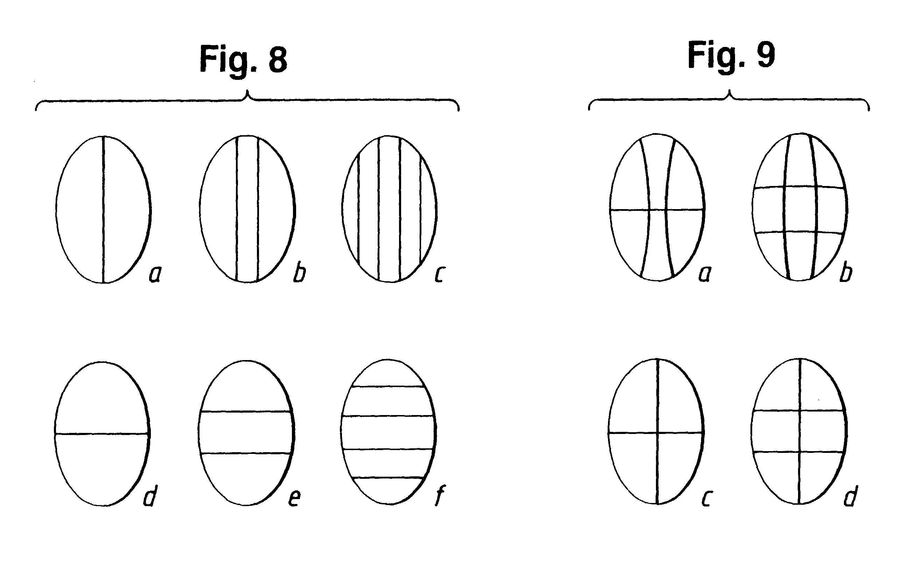

Die Figuren 8 und 9 zeigen Möglichkeiten der Unterteilung (Kammerung) des Kernstücks. Das in den Fig. 8a bis c gezeigte Design betrifft Längskammern und die Ausgestaltungen gemäß Fig. 8d bis f Querkammerungen. In Fig. 9 sind Längs- und Querkammerungen gleichzeitig gezeigt. Die Kammerung gemäß Fig. 8b entspricht der in den Fig. 2 bis 4 gezeigten.

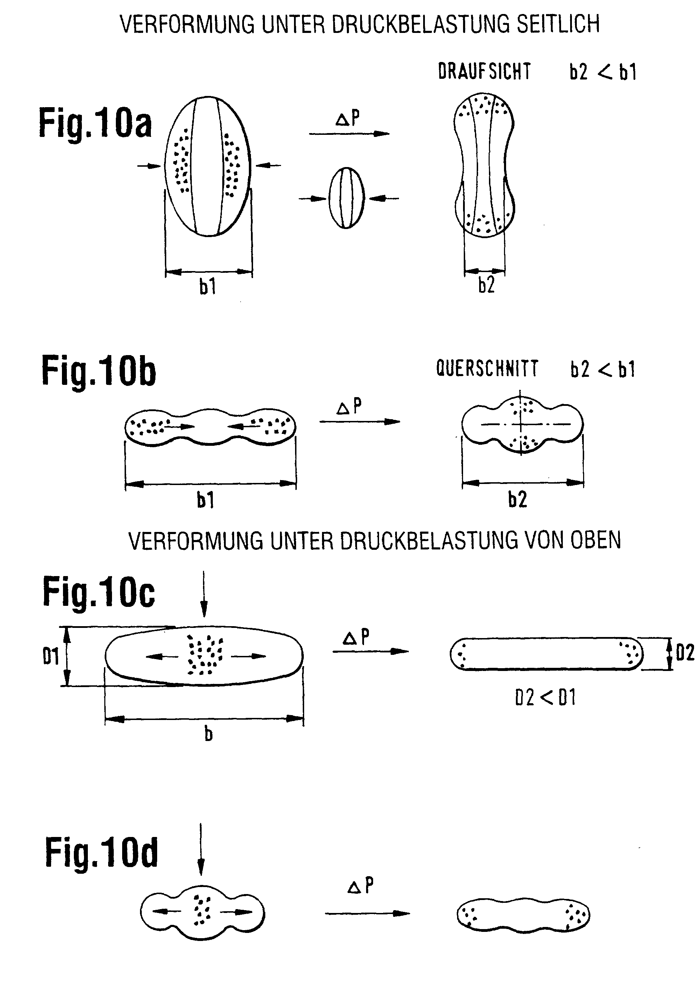

In Fig. 10 sind verschiedene Verformungen des Saugkörpers bei seitlicher Druckbelastung und Druckbelastung von oben aufgezeigt. Ein in Draufsicht im Ausgangszustand ovales Kernstück kann dabei in ein sanduhr- oder hundeknochenförmiges Kernstück übergehen (Fig. 10a), wenn durch die Schenkel der Trägerin seitliche Druckkräfte auf das Kernstück ausgeübt werden (vgl. Pfeile in der linken Abbildung von Fig. 10a), Die ursprüngliche Breite b₁ (linke Abbildung in Fig. 10a) im Schrittbereich vermindert sich dabei auf die Breite b₂ (rechte Abbildung in Fig. 10a), Dadurch wird die besondere "Anschmiegsamkeit" des erfindungsgemäßen absorbierenden Artikels deutlich.

Fig. 10b zeigt die in Fig. 10a beschriebenen Verformungen eines Kernstücks eines absorbierenden Artikels im Querschnitt. Diese Figur zeigt auch deutlich, wie absorbierendes Material aus den beiden seitlich gelegenen Kammern in die zentrale Kammer umgelagert werden kann. Die Pfeile geben die Richtung der Materialwandlung an.

Daß bei seitlicher Druckbelastung sich zwar die äußere Form und die Querschnittskontur des Kernstücks ändern, die Länge l desselben jedoch im wesentlichen unverändert bleibt, ist aus Fig. 10a ersichtlich.

In Fig. 10c ist die Verformung des Saugkörperkernstücks bei Druckbelastung von oben (Pfeil von oben) aufgezeigt. Dabei wird deutlich, wie unter Reduzierung der Dicke des Kernstücks (D2 < D1) Material aus dem zentralen Bereich in seitliche Bereiche umgelagert werden kann (Pfeile nach links und nach rechts). Die Breite b des Kernstücks bleibt dabei im wesentlichen unverändert.

Ist das Kernstück, wie in Fig. 10d gezeigt, gekammert, so wird bei Druckbelastung von oben (Pfeil von oben) die Dicke der mittleren (zentralen) Kammer abnehmen und Material in die seitlich gelegenen Kammern ausweichen, angedeutet durch die Pfeile nach links und rechts, (vergl. auch Fig. 3).

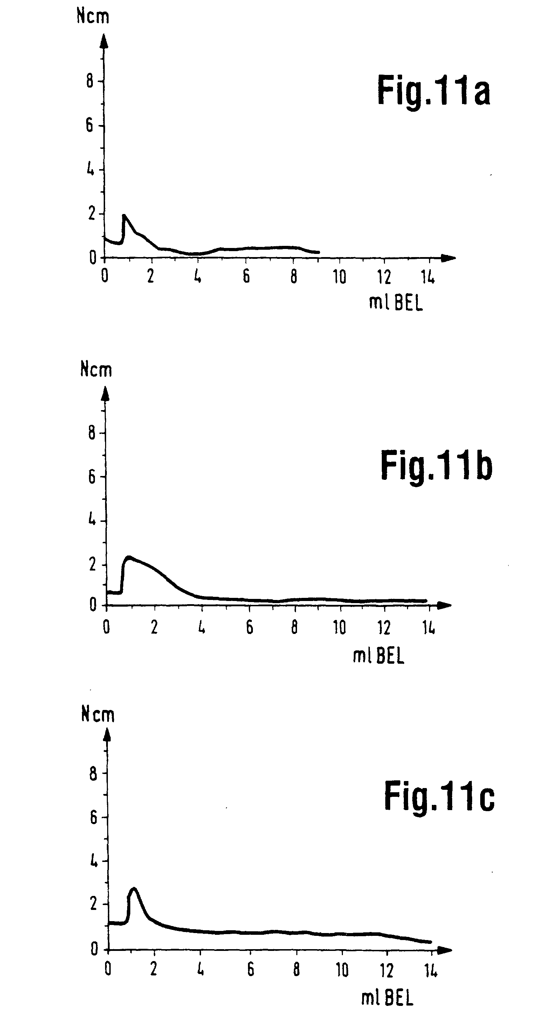

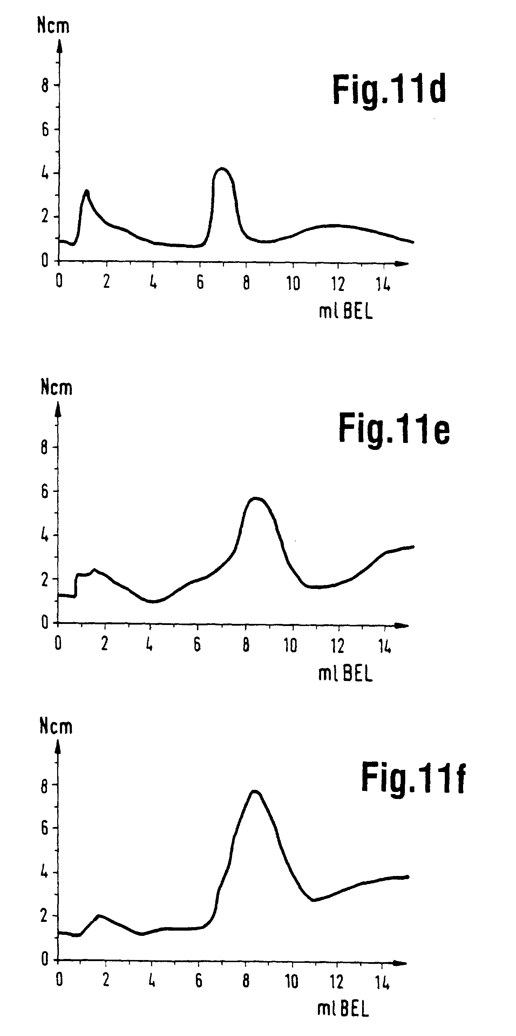

In den Fig. 11e bis f ist das Drehmoment (in Ncm) beim Rühren von Polymethylenharnstoff-Pulver bzw. PMH/Polyacrylat (Superabsorber)-Mischungen bei Zugabe bestimmter Mengen Blutersatzlösung (BEL) angegeben. Fig. 11a beschreibt reines PMH und zeigt ein Maximum von ca. 2 Ncm bei ca. 1 ml BEL. Ähnliche Verhältnisse ergeben sich bei 8 Masseteilen PMH zu 1 Masseteil Polyacrylat (SAP) wie in Fig. 11b dargestellt und bei einer Mischung aus 4 Masseteilen PMH und 1 Masseteil SAP (Fig. 11c), wobei das Maximum bei 1 ml BEL geringfügig ansteigt (Fig. 11b ca. 2,4 Ncm und Fig. 11c ca. 2,8 Ncm).

Untersucht man Mischungen aus 2 Masseteilen PMH und 1 Masseteil SAP, so fällt auf, daß das 1 ml-Maximum auf ca. 3,2 Ncm ansteigt und ein zweites Maximum von ca. 4,2 Ncm bei ca. 7 ml BEL auftritt (Fig. 11d). Bei einer Mischung aus gleichen Masseteilen PMH und SAP findet man ein erstes Maximum von ca. 2,5 Ncm bei etwa 1,8 ml BEL und ein zweites Maximum von ca. 6 Ncm bei ca. 8,3 ml BEL (Fig. 11e). Dieses zweite Maximum steigt bei einer Mischung enthaltend 1 Masseteil PMH und 2 Masseteilen SAP auf ca. 8 Ncm bei ca. 8,3 ml BEL an (Fig. 11f).

Aus den in Fig. 11a bis f gezeigten Daten ist klar ersichtlich, daß (nach Überwindung eines ersten Maximums bei ca. 1 ml Flüssigkeitsbeaufschlagung) reines PMH und Mischungen von bis zu 4 Masseteilen PMH mit 1 Masseteil SAP mit größeren Mengen (bis zu 14 ml) BEL beaufschlagt werden können, ohne daß sich ein wesentlicher Reibungswiderstand der Teilchen des absorbierenden Materials ergibt. Dies ist ein wichtiger Indikator für den hohen Tragekomfort der absorbierenden Artikel, die entsprechende Materialien als Absorbens enthalten. Nachdem, wie vorstehend bereits angedeutet worden war, die meisten Damenbinden mit nicht mehr als 5 ml Flüssigkeit beaufschlagt werden, stört auch das ausgeprägte zweite Maximum nicht, das bei Mischungen auftritt, die weniger als 2 Masseteile PMH pro Masseteil SAP enthalten.

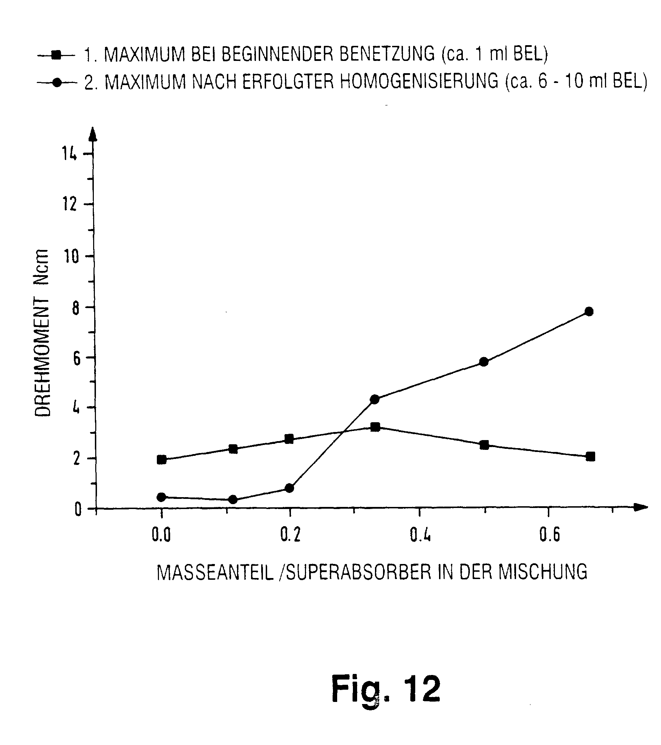

In Fig. 12 schließlich ist das maximale Drehmoment in Ncm bei beginnender Benetzung mit BEL und nach erfolgter Homogenisierung von PMH/SAP-Mischungen angegeben.

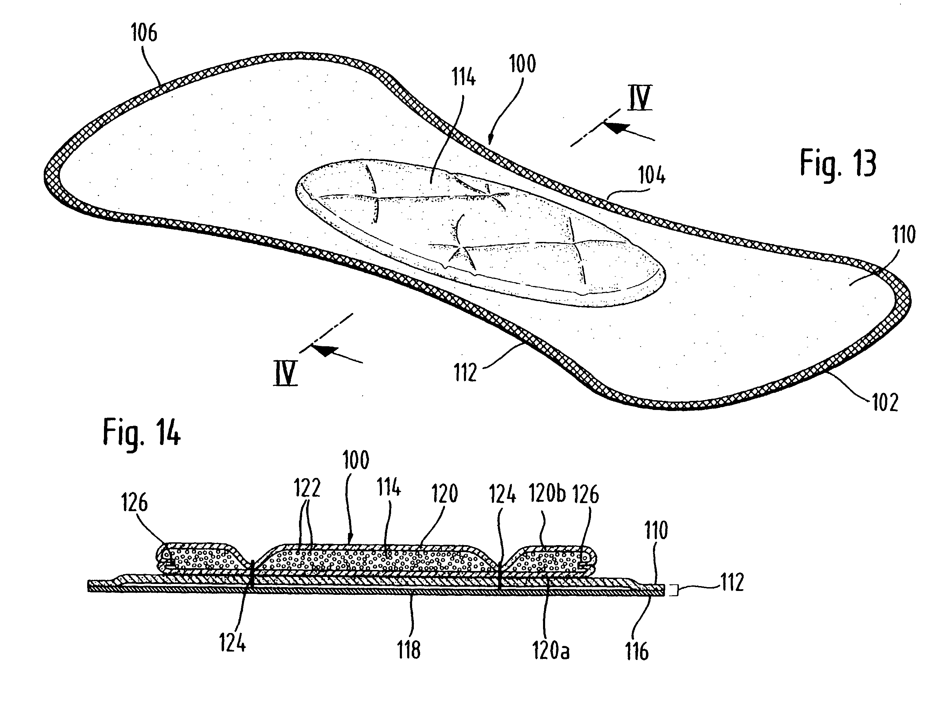

In Fig. 13 ist eine erfindungsgemäße Damenbinde 100 gezeigt, welche einen vorderen Bereich 102, einen mittleren Bereich 104 und einen Endbereich 106 aufweist. Auf einer, in Fig. 13 nicht sichtbaren, flüssigkeitsundurchlässigen Schicht ist 5 eine Schicht 110 aus einem weichen Coformmaterial aufgebracht. Die Schicht 110 ist mit der darunterliegenden flüssigkeitsundurchlässigen Schicht mittels eines Haftmittels 118 verbunden. Im Randbereich sind die beiden Schichten zusätzlich thermisch-mechanisch oder durch Ultraschall miteinander verbunden. Die Schicht 110 dient zum einen dazu, den Tragekomfort des absorbierenden Artikels durch seine Weichheit zu erhöhen; daneben kann die Schicht 110 auch noch als Reserve- oder Sekundärspeicher dienen, wenn aufzunehmende Flüssigkeit in die Randbereiche des absorbierenden Artikels gerät. Auf der Schicht 110 ist ein zentraler Saug- und Speicherkörper 114 angeordnet. Der Saugkörper ist mit einer flüssigkeitsdurchlässigen Umhüllung versehen. In der Umhüllung befindet sich ein flüssigkeitsabsorbierendes Material, welches auch nach Beaufschlagung mit einer Flüssigkeit rieselfähig verbleibt.

Der Aufbau des erfindungsgemäßen absorbierenden Artikels gemäß Fig. 13 ist im Detail in Fig. 14 gezeigt, wobei Fig. 14 einen Querschnitt entlang der in Fig. 13 eingezeichneten Linie IV-IV darstellt. In Fig. 14 ist nun die flüssigkeitsundurchlässige, rückwärtige Abdeckschicht 116 aus Polyethylen gezeigt. Die Abdeckschicht 116 dient einerseits als Auflage für die darüberliegenden, näher zum Körper des Trägers hin angeordneten Schichten des absorbierenden Artikels und andererseits als "Wäscheschutzfolie", welche die Unterbekleidung des Trägers vor Verunreinigungen mit Körperausscheidungen schützt. Im Randbereich 112 sind die flüssigkeitsundurchlässige Schicht 116 und die Schicht 110 aus einem weichen Coformmaterial miteinander verprägt. Auf der Schicht 110 ist der Saugkörper 114 angebracht. Der Saugkörper 114 weist eine Umhüllung 120 aus einem Vliesmaterial auf, in welcher ein auch nach Beaufschlagung mit einer Flüssigkeit rieselfähig verbleibendes Material 122, nämlich Polymethylenharnstoff (PMH) in Kugelform mit einem Teilchendurchmesser im Bereich zwischen 200 und 800 *µ*m. Die Umhüllung 120 ist mittels Nähten 124 mit der Schicht 110 verbunden. Die Nähte 124 bewirken einerseits einen Zusammenhalt der Schicht 110 mit dem Saugkörper 114 und andererseits auch eine gewisse Kompartimentierung des Saugkörpers 114. Dabei wird durch die Nähte 124 bei dem in Fig. 13 und 14 gezeigten Ausführungsbeispiel der Saugkörper nicht in zwei oder mehr komplett voneinander getrennte Bereiche aufgeteilt, da die Nähte nicht durchgängig angeordnet sind. Durch die Art der in Fig. 14 gezeigten Vernähung des Saugkörpers 114 mit der Coformschicht 110 ist auch gewährleistet, daß die darunterliegende, flüssigkeitsundurchlässige Schicht 116 unversehrt bleibt und ihrer Funktion als Wäscheschutzfolie nachkommen kann. Der Saugkörper 114 weist randständige Bereiche 126 auf, an denen ein coformschichtseitiger Abschnitt 120a der Umhüllung 120 mit einem körperseitigen Abschnitt 120b der Umhüllung 120 durch ein Haftmittel miteinander verbunden sind. Vor dem Befüllen des Saugkörpers 114 mit dem saug- und speicherfähigen Material 122 werden die Bereiche 120a und 120b zunächst teilweise miteinander verbunden, derart, daß eine Einfüllöffnung verbleibt. Anschließend wird das Material 122 bis zum gewünschten Befüllungsgrad, im vorliegenden Fall 80% des theoretischen Gesamtfüllvermögens, durch die Einfüllöffnung eingebracht, und anschließend werden die Bereiche 120a und 120b auch im Einfüllöffnungsabschnitt miteinander verbunden, so daß der fertige Saugkörper 114 entsteht, welcher anschließend mittels Nähten auf der Coformschicht 110 fixiert wird.

Der Vorteil des erfindungsgemäßen absorbierenden Artikels 100, wie er in den Fig. 13 und 14 gezeigt ist, gegenüber herkömmlichen absorbierenden Artikeln ist darin zu sehen, daß der Saugkörper und das darin befindliche saugfähige Material einerseits sehr leicht zugänglich sind, da sich zwischen saugfähigem Material und dem Körper des Trägers nur die Umhüllung des Saugkörpers befindet, und andererseits kann durch die freie und exponierte Lage des Saugkörpers dieser sich optimal den anatomischen Gegebenheiten des Trägers anpassen, wodurch der erfindungsgemäße Artikel einen sehr hohen Tragekomfort für den Träger gewährleistet.

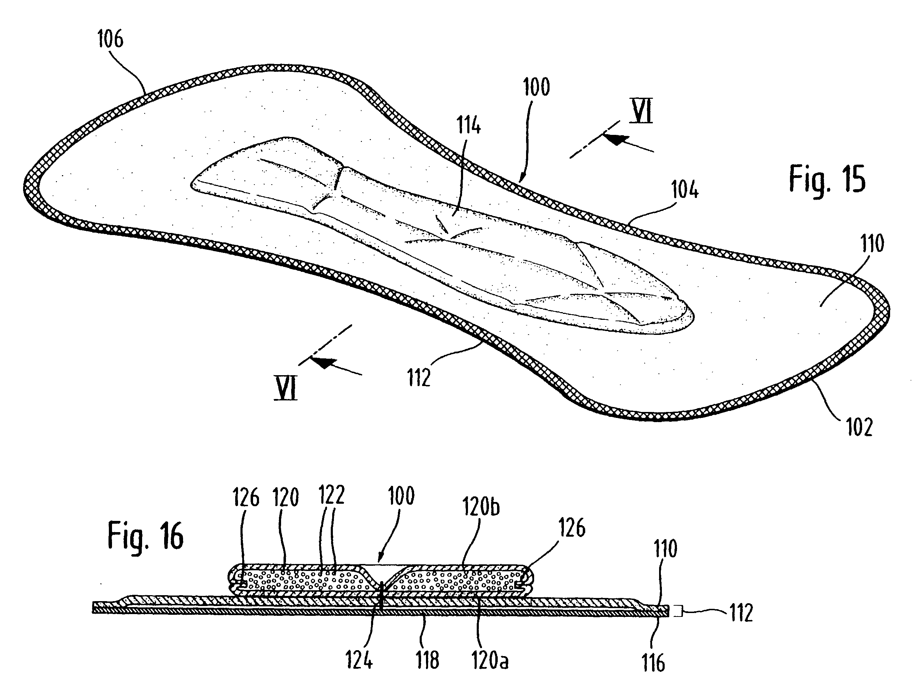

Die Fig. 15 und 16 zeigen ebenfalls eine Damenbinde, welche den prinzipiell gleichen Aufbau wie die in den Fig. 13 und 14 gezeigte Ausführungsform aufweist, wobei die Fig. 16 einen Querschnitt durch den Artikel entlang der Linie VI-VI in Fig. 15 darstellt. Die Bezugszeichen in den Fig. 15 und 16 entsprechen denen in den Fig. 13 und 14. Auch die im Zusammenhang mit den Fig. 13 und 14 beschriebenen Materialien entsprechen denen in Fig. 15 und 16. Wie aus den perspektivischen Ansichten in Fig. 13 und 15 ersichtlich ist, unterscheiden sich die Ausführungsformen gemäß Fig. 13 und 15 in der Form des Saugkörpers 114. Während der Saugkörper 114 gemäß Fig. 13 eine länglich ovale Form aufweist, ist der Saugkörper gemäß Fig. 15 länglich lanzettförmig ausgestaltet. Durch diese Vergrößerung des Saugkörpers wird die Flüssigkeitsaufnahmekapazität des erfindungsgemäßen absorbierenden Artikels weiter erhöht. Wie aus Fig. 16 ersichtlich ist, ist der Saugkörper 114 mit der darunterliegenden Schicht 110 nur mittels einer zentralen Naht 124 verbunden, wodurch sich die Anpassungsfähigkeit des Saugkörpers 114 an verschiedene Tragesituationen noch weiter verbessert.

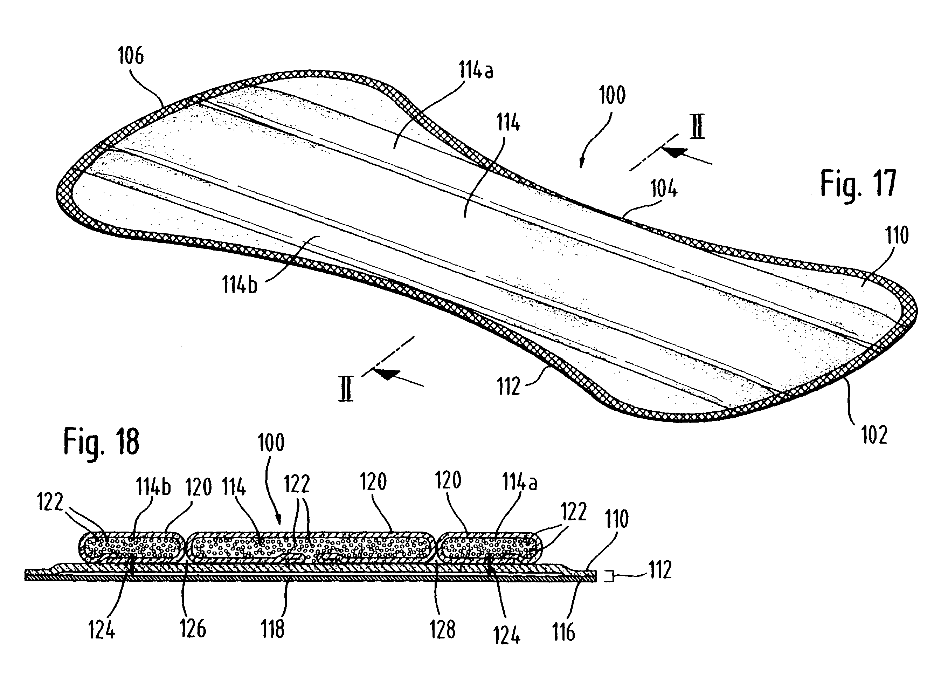

Die Fig. 17 und 18 zeigen schließlich eine weitere Abwandlung des erfindungsgemäßen absorbierenden Artikels, wobei Fig. 17 eine perspektivische Darstellung und Fig. 18 einen Schnitt entlang der Linie II-II in Fig. 17 darstellen. Die in den Fig. 13 und 14 verwendeten Bezugszeichen kennzeichnen entsprechende Bestandteile in den Fig. 17 und 18. Auch die im Zusammenhang mit den Fig. 13 und 14 beschriebenen Materialien entsprechen denen in Fig. 17 und 18.

Die Besonderheit der in den Fig. 17 und 18 gezeigten Ausführungsform eines erfindungsgemäßen absorbierenden Artikels ist die Dreiteilung des Saugkörpers in einen zentralen Abschnitt 114 und zwei seitliche Abschnitte 114a und 114b. Wie aus Fig. 18 ersichtlich ist, weist der Saugkörper drei vollständig voneinander abgeteilte Saugkörperbereiche 114, 114a und 114b auf. Zwischen dem zentralen Saugkörper 114 und den seitlichen Saugkörpern 114a und 114b verlaufen in Längsrichtung Kanäle 126, 128. Diese Ausführungsform des erfindungsgemäßen absorbierenden Artikels gewährleistet einen besonders hohen Auslaufschutz, da bei einem "Überlaufen" des zentralen Saugkörpers 114 noch die seitlichen Saugkörper 114a, 114b zur Flüssigkeitsaufnahme zur Verfügung stehen. Diese Art des absorbierenden Artikels ist daher besonders geeignet für Situationen, in denen große Flüssigkeitsmengen in relativ kurzen Zeitspannen aufgesaugt und gespeichert werden müssen. Dabei unterstützen die Kanäle 126, 128 eine Verteilung der Flüssigkeit in Längsrichtung des absorbierenden Artikels, d.h., die insgesamt zur Verfügung stehende Saug- und Speicherkapazität wird noch besser genutzt, da nahe dem zentralen Beaufschlagungsbereich auch rand- und endständige Bereiche des Saugkörpers optimal zur Flüssigkeitsspeicherung genutzt werden können.

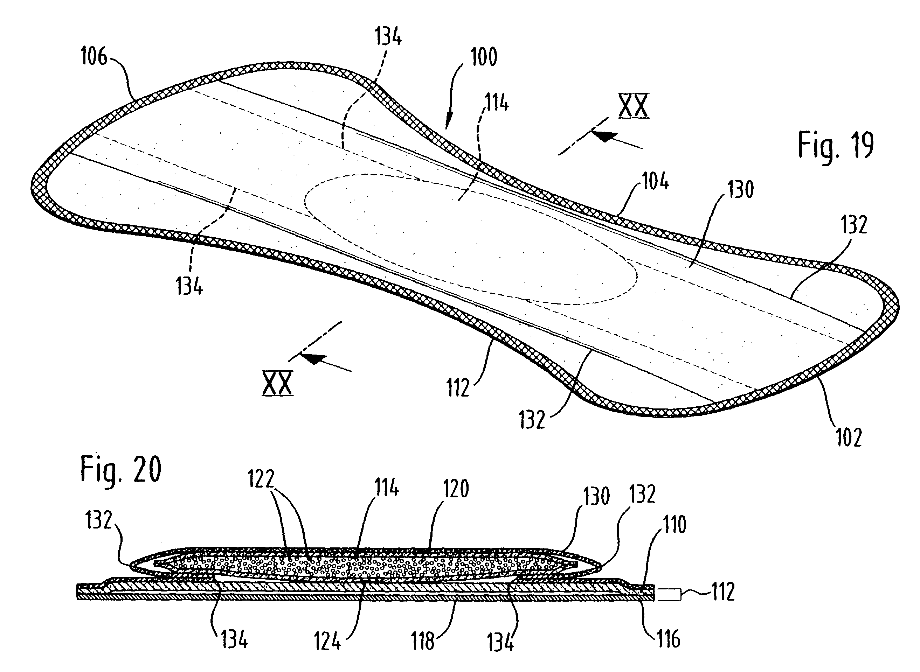

Eine weitere spezielle Ausgestaltung des erfindungsgemäßen absorbierenden Artikels ist in den Fig. 19 und 20 dargestellt. Der Artikel ist wiederum eine Damenbinde, der in Fig. 19 in perspektivischer Ansicht und in Fig. 20 im Querschnitt gezeigt ist. Der Artikel weist eine flüssigkeitsundurchlässige Schicht 116 aus Polyethylen auf, welche beim Gebrauch des Artikels dem Körper des Trägers abgewandt ist. Auf dieser Schicht 116 ist eine Schicht 110 aus weichem Cofommaterial angeordnet, welche einerseits dazu dient, den Tragekomfort des Artikels zu erhöhen. Andererseits kann diese Schicht 110 auch als ein Reserve- oder Sekundärspeicher dienen, welcher nicht in dem Saugkörper 114 aufgenommene und zurückgehaltene Flüssigkeit aufnimmt und speichert.

Der Saugkörper 114 wiederum ist von einer flüssigkeitsdurchlässigen Schicht oder Umhüllung 120 aus Vliesmaterial umgeben. Das flüssigkeitsaufsaugende und -speichernde Material 122 in dem Saugkörper 114 ist ethergruppenfreies und formaldehydgruppenfreies Polymethylenharnstoffmaterial, wobei der Saugkörper zu 70% seiner theoretischen Kapazität mit dem PMH-Material gefüllt ist. Dieser Füllgrad erlaubt eine sehr gute Anpassungsfähigkeit des Artikels an die Anatomie des Trägers.

Der Saugkörper 114 ist über eine Naht oder Verklebung 124 mit der darunterliegenden Schicht 110 und über diese wiederum mit der flüssigkeitsundurchlässigen Schicht 116 verbunden. Mit dem Bezugszeichen 112 ist der randständige, die Schichten 110 und 116 verbindende Bereich gekennzeichnet.

Die Besonderheit des in den Fig. 19 und 20 dargestellten Ausführungsbeispiels ist die Anordnung der flüssigkeitsdurchlässigen Abdeckschicht 130.

Diese Abdeckschicht weist mit dem Bezugszeichen 132 gekennzeichnete Falten auf, die sich in Längsrichtung des Artikels erstrecken. Die Faltung der Schicht 130 läuft dabei bis unter den Saugkörper 114 bis zu einer weiteren Faltung 134, von wo aus die Abdeckschicht sich wieder in Richtung des Randes des Artikels erstreckt. Zwischen der Faltung 134 und dem Randbereich des Artikels ist die Schicht 130 mit der darunterliegenden Schicht 110 mittels Verklebung verbunden. Durch diese spezielle Anordnung der Abdeckschicht 130 wird eine hohe Beweglichkeit und Anpassungsfähigkeit des Saugkörpers und des gesamten Artikels an die Anatomie des Trägers bewirkt.

## Patentansprüche

1. Absorbierender Artikel, mit:
einer bei Verwendung des Artikels dem Körper zugewandten, flüssigkeitsdurchlässigen Schicht (18),
einer bei Verwendung des Artikels dem Körper abgewandten, flüssigkeitsundurchlässigen Schicht (20), sowie
einem zwischen der flüssigkeitsdurchlässigen Schicht (18) und der flüssigkeitsundurchlässigen Schicht (20) angeordneten Saugkörper,
**dadurch gekennzeichnet, daß**
der Saugkörper ein saugfähiges Material (32) enthält, welches auch nach Beaufschlagung mit einer Flüssigkeit rieselfähig bleibt.

2. Absorbierender Artikel, mit
einer bei Verwendung des Artikels dem Körper abgewandten, flüssigkeitsundurchlässigen Schicht (116) sowie einem von einer flüssigkeitsdurchlässigen Schicht umhüllten Saugkörper (114; 114a, 114b), welcher ein auch nach Beaufschlagung mit einer Flüssigkeit rieselfähig bleibendes, saugfähiges Material (122) enthält, wobei der Saugkörper (114; 114a, 114b) mit der flüssigkeitsundurchlässigen Schicht (116) in einem zentralen Bereich derselben verbunden ist.

3. Absorbierender Artikel nach Anspruch 2, **dadurch gekennzeichnet, daß** die Verbindung zwischen Saugkörper (114; 114a, 114b) und flüssigkeitsundurchlässiger Schicht (20; 116) mittels eines Haftmittels bewirkt ist.

4. Absorbierender Artikel nach einem der Ansprüche 2 oder 3, **dadurch gekennzeichnet, daß** die Verbindung zwischen Saugkörper (114; 114a, 114b) und flüssigkeitsundurchlässiger Schicht (11b) mittels einer Naht (124) oder mehrerer Nähte (124) bewirkt ist.

5. Absorbierender Artikel nach einem der vorhergehenden Ansprüche, **dadurch gekennzeichnet, daß** auf der dem Körper zugewandten Seite der flüssigkeitsundurchlässigen Schicht (20; 116) ein als Sekundärspeicher dienendes, saugfähiges, weiches Material (110) angeordnet ist.

6. Absorbierender Artikel nach Anspruch 5, **dadurch gekennzeichnet, daß** das als Sekundärspeicher dienende, weiche Material (110) ein Coformmaterial, ein Airlaidmaterial, Tissuewatte und/oder ein Vliesmaterial, insbesondere Spinnvliese oder Kardenvliese, ist.

7. Absorbierender Artikel nach einem der vorhergehenden Ansprüche, **dadurch gekennzeichnet, daß** das saugfähige Material (32; 122), welches auch nach Beaufschlagung mit einer Flüssigkeit rieselfähig verbleibt, in einer Matrix aus Fasermaterial eingelagert ist.

8. Absorbierender Artikel nach Anspruch 7, **dadurch gekennzeichnet, daß** das saugfähige Material (32; 122), welches auch nach Beaufschlagung mit einer Flüssigkeit rieselfähig verbleibt, in das Fasermaterial homogen eingemischt ist.

9. Absorbierender Artikel nach Anspruch 7 oder 8, **dadurch gekennzeichnet, daß** das saugfähige Material (32; 122), das auch nach Beaufschlagung mit einer Flüssigkeit rieselfähig verbleibt, zwischen Schichten aus Fasermaterial eingelagert ist.

10. Absorbierender Artikel nach einem der Ansprüche 7 bis 9, **dadurch gekennzeichnet, daß** das Fasermaterial Zellstoff, eine Zellstoff/Polypropylen-Mischung und/oder ein Coform-Material ist.

11. Absorbierender Artikel nach einem der Ansprüche 7 bis 10, **dadurch gekennzeichnet, daß** das Verhältnis von saugfähigem Material (32; 122), das auch nach Beaufschlagung mit einer Flüssigkeit rieselfähig verbleibt, zu Fasermaterial von 1 bis 25 Gew.-% zu 99 bis 75 Gew.-% beträgt.

12. Absorbierender Artikel nach Anspruch 11, **dadurch gekennzeichnet, daß** das Verhältnis von saugfähigem Material (32; 122), das auch nach Beaufschlagung mit einer Flüssigkeit rieselfähig verbleibt, zu Fasermaterial von 5 bis 20 Gew.-% zu 95 bis 80 Gew.-% beträgt.

13. Absorbierender Artikel nach Anspruch 12, **dadurch gekennzeichnet, daß** das Verhältnis von saugfähigem Material (32; 122), das auch nach Beaufschlagung mit einer Flüssigkeit rieselfähig verbleibt, zu Fasermaterial von 10 bis 15 Gew.-% zu 90 bis 85 Gew.-% beträgt.

14. Absorbierender Artikel nach einem der vorhergehenden Ansprüche, **dadurch gekennzeichnet, daß** der Saugkörper (114; 114a, 114b) neben dem saugfähigen Material (32; 122), welches auch nach Beaufschlagung mit einer Flüssigkeit rieselfähig verbleibt, mindestens eine pflegende Substanz enthält.

15. Absorbierender Artikel nach Anspruch 14, **dadurch gekennzeichnet, daß** die mindestens eine pflegende Substanz ein Extrakt aus Aloe Vera, Ringelblumen und/oder Kamille ist.

16. Absorbierender Artikel nach einem der Ansprüche 14 oder 15, **dadurch gekennzeichnet, daß** die pflegenden Substanzen in Mikrokapseln eingelagert sind.

17. Absorbierender Artikel nach Anspruch 16, **dadurch gekennzeichnet, daß** die pflegenden Substanzen in die Mikrokapseln derart eingelagert sind, daß die pflegenden Substanzen beim Tragen des absorbierenden Körpers durch die einwirkenden Kräfte und/oder die Körperwärme freigebbar sind.

18. Absorbierender Artikel nach einem der vorhergehenden Ansprüche, **dadurch gekennzeichnet, daß** immobilisiert auf und/oder in dem saugfähigen Material, welches auch nach Beaufschlagung mit einer Flüssigkeit rieselfähig bleibt oder adsorptiv auf dem saugfähigen Material, bakterizide, fungizide und/oder viruzide Substanzen aufgebracht sind.

19. Absorbierender Artikel nach Anspruch 18, **dadurch gekennzeichnet, daß** als bakterizide Substanzen chlorierte Laevulinsäure und/oder Alkyldimethylbenzylammoniumhalogenide aufgebracht sind.

20. Absorbierender Artikel nach einem der vorhergehenden Ansprüche, **dadurch gekennzeichnet, daß** das saugfähige Material (32; 122), welches auch nach Beaufschlagung mit einer Flüssigkeit rieselfähig bleibt, seine Rieselfähigkeit bis zu mindestens 10 ml Flüssigkeit/Gramm Material beibehält.

21. Absorbierender Artikel nach einem der vorhergehenden Ansprüche, **dadurch gekennzeichnet, daß** das saugfähige Material (32; 122) kugelförmige Teilchen enthält.

22. Absorbierender Artikel nach Anspruch 21, **dadurch gekennzeichnet, daß** die kugelförmigen Teilchen einen Durchmesser von 100 bis 2000 µm, insbesondere von 200 bis 800 µm aufweisen.

23. Absorbierender Artikel nach einem der vorhergehenden Ansprüche, **dadurch gekennzeichnet, daß** das saugfähige Material (32; 122) zumindest einen Anteil an Polymethylenharnstoff (PMH) enthält.

24. Absorbierender Artikel nach Anspruch 23, **dadurch gekennzeichnet, daß** mindestens ein Drittel des saugfähigen Materials (32; 122) aus PMH besteht.

25. Absorbierender Artikel nach Anspruch 23, **dadurch gekennzeichnet, daß** mindestens die Hälfte des saugfähigen Materials (32; 122) aus PMH besteht.

26. Absorbierender Artikel nach Anspruch 23, **dadurch gekennzeichnet, daß** mindestens zwei Drittel des saugfähigen Materials (32; 122) aus PMH bestehen.

27. Absorbierender Artikel nach Anspruch 23, **dadurch gekennzeichnet, daß** mindestens 80% des saugfähigen Materials (32; 122) aus PMH besteht.

28. Absorbierender Artikel nach Anspruch 23, **dadurch gekennzeichnet, daß** der Saugkörper aus PMH besteht.

29. Absorbierender Artikel nach einem der Ansprüche 23 bis 28, **dadurch gekennzeichnet, daß** das PMH-Material ethergruppen- und formaldehydfrei ist.

30. Absorbierender Artikel nach einem der Ansprüche 1 bis 27 oder 29, **dadurch gekennzeichnet, daß** das saugfähige Material (32; 122) ein superabsorbierendes Material enthält.

31. Absorbierender Artikel nach Anspruch 30, **dadurch gekennzeichnet, daß** das superabsorbierende Material ein Polyacrylat ist.

32. Absorbierender Artikel nach einem der vorhergehenden Ansprüche, **dadurch gekennzeichnet, daß** der Saugkörper mindestens ein Kernstück (28) aufweist, in welchem das saugfähige Material (32; 122), das auch nach Beaufschlagung mit einer Flüssigkeit rieselfähig bleibt, aufgenommen ist, wobei vorzugsweise die Länge 1 des Kernstücks (28) kleiner oder gleich der Länge L des absorbierenden Artikels und die Breite b des Kernstücks (28) kleiner oder gleich der Breite B des absorbierenden Artikels ist.

33. Absorbierender Artikel nach einem der vorhergehenden Ansprüche, **dadurch gekennzeichnet, daß** der Saugkörper zumindest zwei Kammern (36, 38, 40; 114; 114a, 114b) aufweist, welche mittels mindestens einer Wand voneinander abgetrennt sind.

34. Absorbierender Artikel nach Anspruch 33, **dadurch gekennzeichnet, daß** die zumindest eine Wand in Längsrichtung des absorbierenden Artikels verläuft.

35. Absorbierender Artikel nach Anspruch 33, **dadurch gekennzeichnet, daß** die zumindest eine Wand in Querrichtung des absorbierenden Artikels verläuft.

36. Absorbierender Artikel nach Anspruch 33, **dadurch gekennzeichnet, daß** der Saugkörper durch zumindest eine in Längsrichtung des absorbierenden Artikels verlaufende und zumindest eine weitere in Querrichtung des absorbierenden Artikels verlaufende Wand kompartimentiert ist.

37. Absorbierender Artikel nach einem der Ansprüche 33 bis 36, **dadurch gekennzeichnet, daß** das mindestens eine Kernstück (28) des Saugkörpers in Kammern unterteilt ist.

38. Absorbierender Artikel nach einem der vorhergehenden Ansprüche, **dadurch gekennzeichnet, daß** es ein Hygieneartikel ist.

39. Absorbierender Artikel nach Anspruch 38, **dadurch gekennzeichnet, daß** es ein Damenhygieneartikel ist.

40. Absorbierender Artikel nach Anspruch 39, **dadurch gekennzeichnet, daß** der Damenhygieneartikel eine Damenbinde (10; 100), insbesondere eine ultradünne Damenbinde, ist.

41. Absorbierender Artikel nach Anspruch 39, **dadurch gekennzeichnet, daß** der Damenhygieneartikel eine Slipeinlage ist.

42. Absorbierender Artikel nach einem der Ansprüche 38 bis 41, **dadurch gekennzeichnet, daß** die flüssigkeitsdurchlässige Schicht (18) eine zentral gelegene Öffnung aufweist.

43. Absorbierender Artikel nach einem der Ansprüche 1 bis 38, **dadurch gekennzeichnet, daß** der absorbierende Artikel eine Windel ist.

44. Absorbierender Artikel nach einem der Ansprüche 1 bis 38, **dadurch gekennzeichnet, daß** der absorbierende Artikel eine Inkontinenzeinlage ist.

## Claims

1. An absorbing article, comprising:
a liquid-permeable layer (18) facing the body during use of the article,
a liquid-impermeable layer (20) opposing the body during use of the article, and
an absorbing body provided between the liquid-permeable layer (18) and the liquid-impermeable layer (20),
**characterized in that**
the absorbing body comprises an absorbing material (32) remaining flowable even subsequent to an impingement with a fluid.

2. The absorbing article, comprising:
a liquid-impermeable layer (116) opposing the body during use of the article and an absorbing body (114; 114a, 114b), wrapped by a liquid-permeable layer, said body comprising an absorbing material (112) remaining flowable even subsequent to an impingement with a fluid, wherein the absorbing body (114, 114a, 114b) is connected with the liquid-impermeable layer (116) in a central region thereof.

3. The absorbing article according to claim 2, **characterized in that** the connection between the absorbing body (114; 114a, 114b) and the liquid-impermeable layer (20; 116) is by means of an adhesive.

4. The absorbing article according to one of the claims 2 or 3, **characterized in that** the connection between the absorbing body (114; 114a, 114b) and the liquid-impermeable layer (11b) is by means of a seam (124) or several seams (124).

5. The absorbing article according to one of the preceding claims, **characterized in that** an absorbing, soft material (110), serving as a secondary reservoir, is provided on the liquid-impermeable layer (20; 116), opposing the body.

6. The absorbing article according to claim 5, **characterized in that** the soft material (110), serving as a secondary reservoir, is a coform material, an airlaid material, cotton tissue, and/or a non-woven web, in particular spunbonded web or card web.

7. The absorbing article according to one of the preceding claims, **characterized in that** the absorbing material (32; 122), which remains flowable even subsequent to an impingement with a fluid, is inserted into a matrix made from a fibrous material.

8. The absorbing article according to claim 7, **characterized in that** the absorbing material (32; 122), which remains flowable even subsequent to an impingement with a fluid, is mixed homogeneously with the fibrous material.

9. The absorbing article according to claim 7 or 8, **characterized in that** the absorbing material (32; 122), which remains flowable even subsequent to an impingement with a fluid, is stored between the layers of a fibrous material.

10. The absorbing article according to one of the claims 7 through 9, **characterized in that** the fibrous material is a cellulose, a cellulose/polypropylene mixture, and/or a coform material.

11. The absorbing article according to one of the claims 7 through 10, **characterized in that** the ratio of absorbing material (32; 122), which remains flowable even subsequent to an impingement with a fluid, to the fibrous material is 1 to 25 percent by weight to 99 to 75 percent by weight.

12. The absorbing article according to claim 11, **characterized in that** the ratio of absorbing material (32; 122), which remains flowable even subsequent to an impingement with a fluid, to the fibrous material is 5 to 20 percent by weight to 95 to 80 percent by weight.

13. The absorbing article according to claim 11, **characterized in that** the ratio of absorbing material (32; 122), which remains flowable even subsequent to an impingement with a fluid, to the fibrous material is 10 to 15 percent by weight to 90 to 85 percent by weight.

14. The absorbing article according to one of the preceding claims, **characterized in that** the absorbing body (114; 114a, 114b) comprises at least one care substance in addition to the absorbing material (32; 122), which remains flowable even subsequent to an impingement with a fluid.

15. The absorbing article according to claim 14, **characterized in that** the at least one care substance is an extract of aloe vera, marigold, and/or chamomile.

16. The absorbing article according to on of the claims 14 or 15, **characterized in that** the care substances are incorporated in microcapsules.

17. The absorbing article according to claim 16, **characterized in that** the care substances are incorporated in the micro-capsules such that the care substances are releasable by means of the body heat and/or by impacting forces when the absorbing body is worn.

18. The absorbing article according to one of the preceding claims, **characterized in that** bactericidal, fungicidal and/or virucidal substances are adsorbantly or immobilized applied onto and/or into the absorbing material, which remains flowable even subsequent to an impingement with a fluid.

19. The absorbing article according to claim 18, **characterized in that** chlorinated laevuline acid and/or alkyl dimethylbenzyl ammonium halogenides are applied as bactericidal substances.

20. The absorbing article according to one of the preceding claims, **characterized in that** the absorbing material (32; 122), which remains flowable even subsequent to an impingement with a fluid, keeps its flowablility up to at least 10 ml of fluid/gram of material.

21. The absorbing article according to one of the preceding claims, **characterized in that** the absorbing material (32; 122) comprises spherical particles.

22. The absorbing article according to claim 21, **characterized in that** the spherical particles have a diameter of 100 to 2000 *µ*m, particularly of 200 to 800 *µ*m.

23. The absorbing article according to one of the preceding claims, **characterized in that** the absorbing material (32; 122) comprises at least some polymethylene urea (PMH).

24. The absorbing article according to claim 23, **characterized in that** at least one third of the absorbing material (32; 122) consists of PMH.

25. The absorbing article according to claim 23, **characterized in that** at least half of the absorbing material (32; 122) consists of PMH.

26. The absorbing article according to claim 23, **characterized in that** at least two thirds of the absorbing material (32; 122) consists of PMH.

27. The absorbing article according to claim 23, **characterized in that** at least 80 % of the absorbing material (32; 122) consists of PMH.

28. The absorbing article according to claim 23, **characterized in that** the absorbing body consists of PMH.

29. The absorbing article according to one of the claims 23 through 28, **characterized in that** the PMH-material contains no ether groups or formaldehyde.

30. The absorbing article according to one of the claims 1 through 27 or 29, **characterized in that** the absorbing material (32; 122) comprises a superabsorbing material.

31. The absorbing article according to claim 30, **characterized in that** the superabsorbing material is a polyacrylate.

32. The absorbing article according to one of the preceding claims, **characterized in that** the absorbing body is provided with at least one core (28) accepting the absorbing material (32; 122), which remains flowable even subsequent to an impingement with a fluid, with the length 1 of the core (28) preferably being smaller or equal to the length L of the absorbing article and the width b of the core (28) being smaller or equal to the width B of the absorbing article.

33. The absorbing article according to one of the preceding claims, **characterized in that** the absorbing body is provided with at least two chambers (36, 38, 40; 114; 114a, 114b) being separated from one another by means of at least one wall.

34. The absorbing article according to claim 33, **characterized in that** the at least one wall is directed in the longitudinal direction of the absorbing article.

35. The absorbing article according to claim 33, **characterized in that** the at least one wall is directed in the crosswise direction of the absorbing article.

36. The absorbing article according to claim 33, **characterized in that** the absorbing body is segmented by at least one wall being directed in the longitudinal direction of the absorbing article and another one being directed in the crosswise direction of the absorbing article.

37. The absorbing article according to one of the claims 33 through 36, **characterized in that** the at least one core (28) of the absorbing body is divided into chambers.

38. The absorbing article according to one of the preceding claims, **characterized in that** it is a hygiene product.

39. The absorbing article according to claim 38, **characterized in that** it is a feminine hygiene product.

40. The absorbing article according to claim 39, **characterized in that** the feminine hygiene product is a feminine pad (10; 100), particularly an ultrathin feminine pad.

41. The absorbing article according to claim 39, **characterized in that** the feminine hygiene product is a pantiliner.

42. The absorbing article according to one of the claims 38 through 41, **characterized in that** the liquid-permeable layer (18) is provided with a centrally positioned opening.

43. The absorbing article according to one of the claims 1 through 38, **characterized in that** the absorbing article is a diaper.

44. The absorbing article according to one of the claims 1 through 38, **characterized in that** the absorbing article is an incontinence pad.

## Revendications

1. Article absorbant, ayant:
une couche perméable aux liquides (18) sur la face orientée vers le corps lors de l'utilisation de l'article,
une couche imperméable aux liquides (20) sur la face opposée au corps lors de l'utilisation de l'article, ainsi que
un corps d'absorption disposé entre la couche perméable aux liquides (18) et la couche imperméable aux liquides (20),
**caractérisé en ce que**
le corps d'absorption contient une matière absorbante (32) qui conserve, même après une charge en liquide, sa faculté d'écoulement.

2. Article absorbant, ayant:
une couche imperméable aux liquides (116) sur la face opposée au corps lors de l'utilisation de l'article, ainsi qu'un corps d'absorption (114, 114a, 114b) enveloppé d'une couche perméable aux liquides et contenant une matière absorbante (122) qui conserve, même après une charge en liquide, sa faculté d'écoulement, le corps d'absorption (114, 114a, 114b) étant relié à la couche imperméable aux liquides (116) au niveau d'une zone centrale de celle-ci.

3. Article absorbant selon la revendication 2, **caractérisé en ce que** la jonction entre le corps d'absorption (114, 114a, 114b) et la couche imperméable aux liquides (20, 116) se fait au moyen d'un agent adhésif.

4. Article absorbant selon l'une des revendications 2 et 3, **caractérisé en ce que** la jonction entre le corps d'absorption (114; 114a, 114b) et la couche imperméable aux liquides (11b) se fait au moyen d'une couture (124) ou de plusieurs coutures (124).

5. Article absorbant selon l'une des revendications précédentes, **caractérisé en ce qu'**une matière douce et absorbante (110) servant de réservoir secondaire est disposée sur la face orientée vers le corps de la couche imperméable aux liquides (20; 116).

6. Article absorbant selon la revendication 5, **caractérisé en ce que** la matière douce (110) servant de réservoir secondaire est une matière coformée, une matière étalée par voie sèche, du coton ouaté et/ou une matière non tissée, en particulier une nappe obtenue par filage-nappage ou une nappe cardée.

7. Article absorbant selon l'une des revendications précédentes, **caractérisé en ce que** la matière absorbante (32, 122) conservant, même après une charge en liquide, sa faculté d'écoulement est incorporée dans une matrice de matériau en fibres.

8. Article absorbant selon la revendication 7, **caractérisé en ce que** la matière absorbante (32, 122) conservant, même après une charge en liquide, sa faculté d'écoulement, est mélangée de façon homogène au matériau en fibres.

9. Article absorbant selon la revendication 7 ou 8, **caractérisé en ce que** la matière absorbante (32, 122), conservant, même après une charge en liquide, sa faculté d'écoulement, est incorporée entre des couches du matériau en fibres.

10. Article absorbant selon l'une des revendications 7 à 9, **caractérisé en ce que** le matériau en fibres est de la cellulose, un mélange de cellulose/polypropylène et/ou une matière coformée.

11. Article absorbant selon l'une des revendications 7 à 10, **caractérisé en ce que** le rapport entre la matière absorbante (32, 122) conservant, même après une charge en liquide, sa faculté d'écoulement et le matériau en fibres est de 1 - 25 % en poids à 99 - 75 % en poids.

12. Article absorbant selon la revendication 11, **caractérisé en ce que** le rapport entre la matière absorbante (32, 122) conservant, même après une charge en liquide, sa faculté d'écoulement et le matériau en fibres est de 5 - 20 % en poids à 95 - 80 % en poids.

13. Article absorbant selon la revendication 12, **caractérisé en ce que** le rapport entre la matière absorbante (32, 122) conservant, même après une charge en liquide, sa faculté d'écoulement et le matériau en fibres est de 10 - 15 % en poids à 90 - 85 % en poids.

14. Article absorbant selon l'une des revendications précédentes, **caractérisé en ce que** le corps d'absorption (114; 114a, 114b) contient, en plus de la matière absorbante (32, 122) conservant même après une charge en liquide, sa faculté d'écoulement, au moins une substance de soin.

15. Article absorbant selon la revendication 14, **caractérisé en ce que** la substance de soin est un extrait d'aloès vera, de soucis et/ou de camomille.

16. Article absorbant selon l'une des revendications 14 et 15, **caractérisé en ce que** les substances de soin sont incorporées dans des microcapsules.

17. Article absorbant selon la revendication 16, **caractérisé en ce que** les substances de soin sont incorporées dans les microcapsules de façon telle que, lorsque l'article absorbant est porté, les substances de soin peuvent être libérées par les forces agissantes et/ou par la chaleur du corps.

18. Article absorbant selon l'une des revendications précédentes, **caractérisé en ce que** des substances bactéricides, fongicides et/ou virucides sont immobilisées sur et/ou dans la matière absorbante ou adsorbées sur la matière absorbante conservant, même après une charge en liquide, sa faculté d'écoulement.

19. Article absorbant selon la revendication 18, **caractérisé en ce que** de l'acide lévulique chloré et/ou des halogénures d'alcoyldiméthylbenzylammonium sont appliqués en tant que substances bactéricides.

20. Article absorbant selon l'une des revendications précédentes, **caractérisé en ce que** la matière absorbante (32, 122) conservant, même après une charge en liquide, sa faculté d'écoulement maintient sa faculté d'écoulement jusqu'à 10 ml de liquide/gramme de matière au minimum.

21. Article absorbant selon l'une des revendications précédentes, **caractérisé en ce que** la matière absorbante (32, 122) contient des particules en forme de billes.

22. Article absorbant selon la revendication 21, **caractérisé en ce que** les particules en forme de billes ont un diamètre de 100 à 2000 *µ*m, en particulier de 200 à 800 *µ*m.

23. Article absorbant selon l'une des revendications précédentes, **caractérisé en ce que** la matière absorbante (32, 122) est constituée au moins en partie de polyméthyléne-urée (UPM).

24. Article absorbant selon la revendication 23, **caractérisé en ce qu'**au moins un tiers de la matière absorbante (32, 122) est constitué d'UPM.

25. Article absorbant selon la revendication 23, **caractérisé en ce qu'**au moins la moitié de la matière absorbante (32, 122) est constituée d'UPM.

26. Article absorbant selon la revendication 23, **caractérisé en ce qu'**au moins deux tiers de la matière absorbante (32, 122) sont constitués d'UPM.

27. Article absorbant selon la revendication 23, **caractérisé en ce qu'**au moins 80 % de la matière absorbante (32, 122) est constitué d'UPM.

28. Article absorbant selon la revendication 23, **caractérisé en ce que** le corps d'absorption est constitué d'UPM.

29. Article absorbant selon l'une des revendications 23 à 28, **caractérisé en ce que** le matériau UPM est exempt de groupes d'éther et de formaldéhyde.

30. Article absorbant selon l'une des revendications 1 à 27 ou 29, **caractérisé en ce que** la matière absorbante (32, 122') contient une matière superabsorbante.

31. Article absorbant selon la revendication 30, **caractérisé en ce que** la matière superabsorbante est un polyacrylate.

32. Article absorbant selon l'une des revendications précédentes, **caractérisé en ce que** le corps d'absorption présente au moins un noyau (28) dans lequel est logée la matière absorbante (32, 122) conservant, même après une charge en liquide, sa faculté d'écoulement, la longueur l du noyau (28) étant de préférence inférieure ou égale à la longueur L de l'article absorbant et la largeur b du noyau (28) étant inférieure ou égale à la largeur B de l'article absorbant.

33. Article absorbant selon l'une des revendications précédentes, **caractérisé en ce que** le corps d'absorption présente au moins deux chambres (36, 38, 40, 114, 114a, 114b) séparées l'une de l'autre au moyen d'au moins une paroi.

34. Article absorbant selon la revendication 33, **caractérisé en ce qu'**au moins une paroi se trouve dans le sens de la longueur de l'article absorbant.

35. Article absorbant selon la revendication 33, **caractérisé en ce qu'**au moins une paroi se trouve dans le sens transversal de l'article absorbant.

36. Article absorbant selon la revendication 33, **caractérisé en ce que** le corps d'absorption est compartimenté par au moins une paroi s'étendant dans le sens longitudinal de l'article absorbant et au moins une autre paroi s'étendant dans le sens transversal de l'article absorbant.

37. Article absorbant selon l'une des revendications 33 à 36, **caractérisé en ce qu'**un noyau (28) au moins du corps d'absorption est divisé en chambres.

38. Article absorbant selon l'une des revendications précédentes, **caractérisé en ce qu'**il s'agit d'un article d'hygiène.

39. Article absorbant selon la revendication 38, **caractérisé en ce qu'**il s'agit d'un article d'hygiène féminine.

40. Article absorbant selon la revendication 39, **caractérisé en ce que** l'article d'hygiène féminine est une serviette hygiénique (10, 100), en particulier une serviette hygiénique ultramince.

41. Article absorbant selon la revendication 39, **caractérisé en ce que** l'article d'hygiène féminine est un protège-slip.

42. Article absorbant selon l'une des revendications 38 à 41, **caractérisé en ce que** la couche perméable aux liquides (18) présente un orifice au milieu.

43. Article absorbant selon l'une des revendications 1 à 38, **caractérisé en ce que** l'article absorbant est une couche pour bébés.

44. Article absorbant selon l'une des revendications 1 à 38, **caractérisé en ce que** l'article absorbant est une protection pour incontinents.
